(19) Europäisches Patentamt
European Patent Office
Office européen des brevets

(11) Veröffentlichungsnummer : **0 133 225**
**B1**

(12) # EUROPÄISCHE PATENTSCHRIFT

(45) Veröffentlichungstag der Patentschrift : **19.10.88**

(51) Int. Cl.[4] : **C 07 K 1/00**, A 61 K 37/02

(21) Anmeldenummer : **84108022.9**

(22) Anmeldetag : **09.07.84**

(54) **Neue Peptidderivate und Salze davon, diese enthaltende pharmazeutische Präparate, Verfahren und Zwischenprodukte zu ihrer Herstellung.**

(30) Priorität : **12.07.83 GB 8318855**
**08.05.84 GB 8411635**

(43) Veröffentlichungstag der Anmeldung :
**20.02.85 Patentblatt 85/08**

(45) Bekanntmachung des Hinweises auf die Patenterteilung : **19.10.88 Patentblatt 88/42**

(84) Benannte Vertragsstaaten :
**AT BE CH DE FR GB IT LI LU NL SE**

(56) Entgegenhaltungen :
**CHEMICAL ABSTRACTS, Band 89, Nr. 17, 23. Oktober 1978, Columbus, Ohio, USA; E.A. PORUNKEVICH et al. "Model of the structural-functional organization of adrenocorticotropic hormone", Seite 86, Spalte 1, Zusammenfassungsnr. 140824j**
**CHEMICAL ABSTRACTS, Band 101, Nr. 15, 8. Oktober 1984, Columbus, Ohio, USA; D.B. RICHARDS et al. "Effect of alpha-MSH 11-13 (lysine-proline-valine) on fever in the rabbit", Seite 118, Spalte 1, Zusammenfassungsnr. 123621q & Peptides (Fayetteville, N.Y.) Band 5, Nr. 4, 198**
**G.R. PETTIT "Synthetic Petpides" 1980, ELSEVIER SCIENTIFIC PUBLISHING COMPANY, Amsterdam-Oxford-New York Band 5, Seite 192**
**G.R. PETTIT "Synthetic Peptides" VAN NOSTRAND REINHOLD COMPANY, New York-Cincinnati, Toronto, London, Melbourne Band 1, Seite 210**
**Die Akte enthält technische Angaben, die nach dem Eingang der Anmeldung eingereicht wurden und die nicht in dieser Patentschrift enthalten sind.**

(73) Patentinhaber : **F. HOFFMANN-LA ROCHE & CO. Aktiengesellschaft**
**CH-4002 Basel (CH)**

(72) Erfinder : **Hassall, Cedric Herbert**
**6 Ashcroft Close**
**Harpenden, Herts (GB)**
Erfinder : **Johnson, William Henry**
**87 West Hill**
**Hitchin, Herts. (GB)**
Erfinder : **Roberts, Noel Allan**
**19 Elliswick Road**
**Harpenden, Hrts. (GB)**

(74) Vertreter : **Lederer, Franz, Dr. et al**
**Van der Werth, Lederer & Riederer Patentanwälte**
**Lucile-Grahn-Strasse 22**
**D-8000 München 80 (DE)**

**EP 0 133 225 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft Peptidderivate, ein Verfahren zu deren Herstellung und pharmazeutische Präparate enthaltend diese Peptidderivate. Die Erfindung betrifft weiterhin neue Zwischenprodukte, die in der Herstellung dieser Peptidderivate verwendet werden.

Die Peptidderivate gemäss vorliegender Erfindung sind Verbindungen der allgemeinen Formel

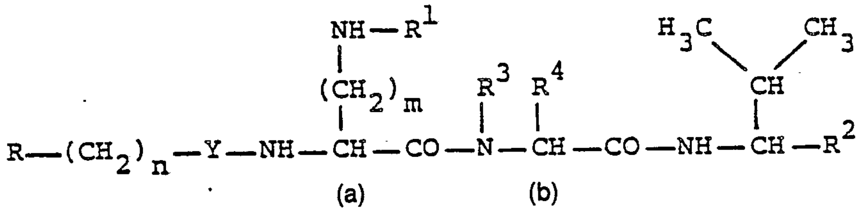

(I)

worin R eine cyclische Kohlenwasserstoffgruppe von 6-12 C-Atomen bedeutet, $R^1$ ein von einer gegebenenfalls durch Amino oder eine oder mehrere Hydroxygruppen substituierten aliphatischen oder aromatischen Dicarbonsäure abgeleiteter Acylrest ist, oder ein Acylrest einer Uronsäure bedeutet, $R^2$ Wasserstoff oder Formyl bedeutet, $R^3$ Wasserstoff und $R^4$ Methyl bedeutet oder $R^3$ und $R^4$ gemeinsam eine Trimethylengruppe bedeuten, Y eine Carbonyl-, Sulfonyl- oder Methylengruppe bedeutet, m für eine ganze Zahl von 2-6 steht und n 0, 1 oder 2 bedeutet, und wobei die (a) und (b) bezeichneten C-Atome die L-Konfiguration aufweisen, sowie pharmazeutisch annehmbare Salze davon.

Die als R bezeichnete cyclische Kohlenwasserstoffgruppe kann eine Vielzahl von Bedeutungen haben. So z. B. kann R eine Phenylgruppe sein oder eine Phenylgruppe, die durch eine oder mehrere Alkylgruppen mit bis zu 4 Kohlenstoffatomen (z. B. Methyl, Aethyl, n-Propyl, Isopropyl, n-Butyl, Isobutyl, etc.), substituiert ist, mit der Massgabe, dass die Gesamtzahl der C-Atome in einer solchen Alkyl substituierten Phenylgruppe 12 nicht übersteigt. Beispiel solcher Alkyl substituierter Phenylgruppen sind o-Tolyl, m-Tolyl, p-Tolyl, 2,4-Dimethylphenyl, etc. Ein weiteres Beispiel einer cyclischen Kohlenwasserstoffgruppe für den Substituenten R ist eine Phenylgruppe, die einen Phenylsubstituenten trägt, wie z. B. 4-Biphenylyl. Weiterhin kann R z. B. eine ankondensierte cyclische Kohlenwasserstoffgruppe wie Naphthyl, Tetrahydronaphthyl, Decahydronaphthyl, Indanyl und dergleichen bedeuten. Zudem kann R eine monocyclische oder verknüpfte Cycloalkylgruppe, besonders Cyclooctyl, Cyclodecyl, Adamantyl, Bicycloheptyl und dergleichen bedeuten.

Wenn $R^1$ eine von einer gegebenenfalls substituierten aliphatischen Dicarbonsäure abgeleitete Acylgruppe bedeutet, so kann diese Acylgruppe von einer gesättigten oder ungesättigten aliphatischen Dicarbonsäure abgeleitet sein, z. B. Bernsteinsäure, Glutarsäure, Adipinsäure, Glutaminsäure, Asparaginsäure, α-Aminoadipinsäure, Aepfelsäure, Weinsäure, Fumarsäure und dergleichen. Beispiele von Acylgruppen $R^1$, die von einer aromatischen Dicarbonsäure abgeleitet sind, sind Acylgruppen, die von einer Benzoldicarbonsäure abgeleitet sind, wie Phthalsäure, Isophthalsäure, Terephthalsäure, etc. oder von einer Naphthalindicarbonsäure wie 1,5-Naphthalindicarbonsäure, 1,8-Naphthalindicarbonsäure, etc. Beispiele von Acylgruppen $R^1$, die von einer Uronsäure hergeleitet sind, sind Acylgruppen, welche von D-Glucuronsäure und D-Galacturonsäure abgeleitet sind.

Wenn $R^2$ in Formel I eine Formylgruppe bedeutet, hat das C-Atom, an welches diese Formylgruppe gebunden ist, die D- oder L-Konfiguration. Die vorliegende Erfindung umfasst solche D- und L-Verbindungen wie Mischungen davon.

Verbindungen der Formel I können pharmazeutisch annehmbare Salze mit Basen bilden, z. B. Alkalimetallsalze, wie Natrium- oder Kaliumsalze und Erdalkalimetallsalze, wie Calcium- und Magnesiumsalze. Verbindungen der Formel I, welche eine freie Aminosäure enthalten, können pharmazeutisch annehmbare Salze mit Säuren bilden. Diese Säuren können anorganische Säuren wie Halogenwasserstoffsäuren (z. B. Chlorwasserstoffsäure oder Bromwasserstoffsäure), Schwefelsäure, Phosphorsäure, Salpetersäure, etc. oder organische Säuren wie z. B. Essigsäure, Maleinsäure, Fumarsäure, Weinsäure, Zitronensäure, Methansulfonsäure und dergleichen, sein.

Bevorzugte Verbindungen der Formel I sind solche, in denen unabhängig voneinander, R eine monocyclische oder verknüpfte Cycloalkylgruppe enthaltend 6-12 C-Atome, bevorzugt Adamantyl bedeutet, $R^1$ die von einer unsubstituierten gesättigten aliphatischen Dicarbonsäure mit bis zu 6 C-Atomen abgeleitete Acylgruppe bedeutet, besonders Succinyl oder Adipolyl oder $R^1$ eine Acylgruppe abgeleitet von einer unsubstituierten Benzoldicarbonsäure, bevorzugt 4-Carboxybenzolsäure bedeutet, $R^2$ eine Formylgruppe bedeutet, Y eine Sulfonylgruppe bedeutet, m für 4 steht und n 0 bedeutet.

Besonders bevorzugte Verbindungen der Formel I sind :

$N^{\alpha}$-(1-Adamantylsulfonyl)-$N^{\varepsilon}$-succinyl-L-lysyl-L-prolyl-L-valinal
$N^{\alpha}$-(1-Adamantylsulfonyl)-$M^{\varepsilon}$-(4-carboxybenzoyl)-L-lysyl-L-prolyl-L-valinal und
$N^{\alpha}$-(1-Adamantylsulfonyl)-$N^{\varepsilon}$-(4-carboxybenzoyl)-L-lysyl-L-alanyl-L-valinal.

Beispiele anderer Verbindungen der Formel I sind :

$N^{\alpha}$-(1-Adamantyläthyl)-$N^{\varepsilon}$-succinyl-L-lysyl-L-prolin-isobutylamid,
$N^{\alpha}$-(6,6-Dimethylbicyclo [3.1.1] heptyläthyl)-$N^{\varepsilon}$-($\gamma$-glutamyl)-L-lysyl-L-prolin-isobutylamid,
$N^{\alpha}$-(1-Adamantanacetyl)-$N^{\varepsilon}$-succinyl-L-lysyl-L-prolin-isobutylamid,
$N^{\alpha}$-(1-Adamantylsulfonyl)-$N^{\varepsilon}$-succinyl-L-lysyl-L-alanylvalinal,
$N^{\alpha}$-(1-Adamantylsulfonyl)-$N^{\varepsilon}$-(2-carboxybenzoyl)-L-lysyl-L-prolylvalinal und
$N^{\alpha}$-(1-Adamantylsulfonyl)-$N^{\varepsilon}$-adipolyl-L-lysyl-L-prolinvalinal.

Nach dem Verfahren gemäss vorliegender Erfindung können die Verbindungen der Formel I und die pharmazeutisch annehmbaren Salze davon erhalten werden, durch

(a) Reaktion einer Verbindung der allgemeinen Formel

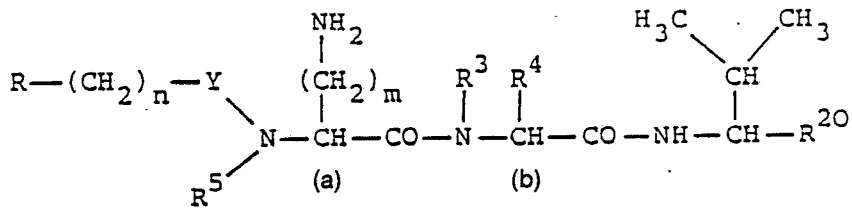

(II)

worin R, $R^3$, $R^4$, Y, m und n die früher angegebene Bedeutung haben, die mit (a) und (b) bezeichneten Kohlenstoffatome die früher angegebene Konfiguration haben, $R^5$ Wasserstoff bedeutet, wenn Y eine Carbonyl- oder Sulfonylgruppe bedeutet oder eine Schutzgruppe bedeutet, wenn Y eine Methylengruppe ist und $R^{20}$ ein Wasserstoffatom oder eine geschützte Formylgruppe bedeutet, mit einem reaktiven Derivat einer Säure der allgemeinen Formel

$$HO—R^{10} \qquad (III)$$

worin $R^{10}$ die gleiche Bedeutung hat wie $R^1$, worin jedoch jede Carboxy-, Amino- oder Hydroxygruppe in geschützter Form vorliegt, und falls erforderlich, Abtrennen allfälliger Schutzgruppen im Reaktionsprodukt oder

(b) zur Herstellung einer Verbindung der Formel I, in der $R^1$ 2-Carboxybenzoyl bedeutet, durch Behandeln einer Verbindung der allgemeinen Formel

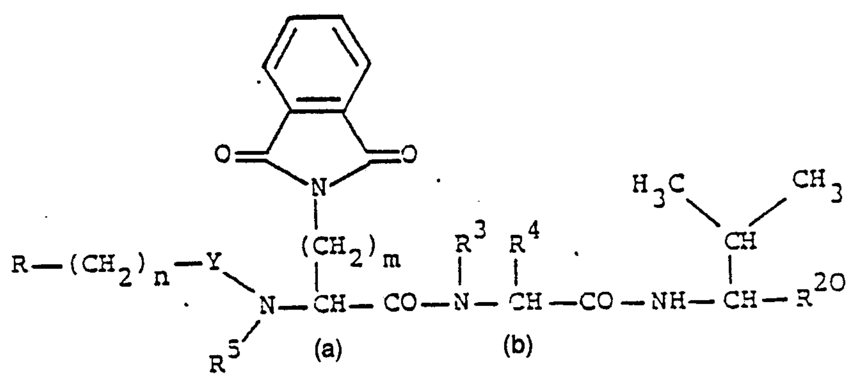

(IV)

worin R, $R^3$, $R^4$, $R^5$, $R^{20}$, Y, m und n die früher angegebene Bedeutung haben und die mit (a) und (b) bezeichneten C-Atomen die früher angegebene Konfiguration haben, mit einem Alkalimetallhydroxyd und, falls erforderlich, Abspaltung von allfälligen Schutzgruppen im Reaktionsprodukt, wobei

(c) in beiden Fällen, sofern erwünscht, eine erhaltene Verbindung der Formel I in ein pharmazeutisch annehmbares Salz überführt werden kann.

Die als $R^5$ bezeichnete Schutzgruppe in Formel II und IV kann eine herkömmliche Schutzgruppe wie z. B. eine tert.-Butoxycarbonyl, Benzyloxycarbonyl, Formyl, Trityl, Trifluoracetyl, 2-(Biphenylyl)-isopropoxycarbonyl oder eine ähnliche Gruppe sein. Vorzugsweise bedeutet $R^5$ eine Benzyloxycarbonylgruppe.

Eine mit $R^{20}$ bezeichnete geschützte Formylgruppe in Formel II und IV ist vorzugsweise eine Formylgruppe, die in Form des Dimethyl- oder Diäthylacetals geschützt ist, kann aber auch eine Formylgruppe sein, die in Form eines Oxims, Semicarbazides oder dergleichen geschützt ist.

Eine Carboxy-, Amino- oder Hydroxygruppe $R^{10}$ einer Säure der Formel III kann in herkömmlicher Weise geschützt sein. So z. B. kann eine Carboxygruppe in Form eines leicht abspaltbaren Esters, wie des Methyl, Aethyl, tert. Butyl, Benzyl oder eines ähnlichen Esters geschützt sein. Eine Aminogruppe kann in ähnlicher Weise geschützt sein, wie $R^5$ in Formel II. Eine Hydroxygruppe kann z. B. geschützt sein in Form eines leicht spaltbaren Aethers, wie des tert.-Butyl- oder Tetrahydropyranyläthers oder in der Form eines leicht spaltbaren Esters wie eines Esters von einer Alkancarbonsäure (z. B. Essigsäure).

Das reaktionsfähige Derivat einer Säure der Formel III kann z. B. ein Säurehalogenid (z. B. das Säurechlorid), ein Säureanhydrid, ein Säureazid, ein aktives Amid (z. B. ein Amid mit Pyrazol, Imidazol, etc.) ein gemischtes Carbonsäureanhydrid oder ein aktiver Ester (z. B. der Cyanomethyl-, 4-Nitrophenyl- oder N-Hydroxybernsteinsäureester) sein.

Die Reaktion einer Verbindung der allgemeinen Formel II mit einem reaktiven Derivat einer Säure der Formel III gemäss Verfahrensaspekt (a) kann in an sich bekannter Weise durchgeführt werden. Die Reaktion wird zweckmässigerweise in einem inerten organischen Lösungsmittel wie einem halogenierten Kohlenwasserstoff, z. B. Methylenchlorid, Chloroform, etc.), einem Aether (z. B. Tetrahydrofuran, Dioxan, etc.), einem Ester (z. B. Essigester, etc.), Acetonitril, Dimethylformamid, Dimethylacetamid, etc. oder in einer Mischung dieser Lösungsmittel durchgeführt. Es ist zweckmässig die Reaktion bei einer Temperatur zwischen — 20 und ungefähr + 40 °C (bevorzugt zwischen ungefähr — 10 °C und ungefähr + 30 °C, und besonders bevorzugt zwischen ungefähr 0 °C und Raumtemperatur durchzuführen. Die Reaktion wird zweckmässigerweise in Gegenwart einer Base, wie eines tertiären organischen Amins (z. B. Triäthylamin, N-Methylmorpholin, N-Aethylmorpholin, etc.) oder eines Alkalimetallcarbonates (z. B. Natriumbicarbonat, etc.) durchgeführt. Unter gewissen Umständen kann es zweckmässig sein, die Reaktion in situ durchzuführen, d. h. ohne Isolierung der Verbindung der Formel II vom Medium, in welchem sie hergestellt wird.

Nach der Reaktion einer Verbindung der Formel II mit einem reaktiven Derivat einer Säure der Formel III werden alle vorhandenen Schutzgruppen im Reaktionsprodukt abgespalten. Die Abspaltung der Schutzgruppen kann in an sich bekannter Weise durchgeführt werden. Schutzgruppen, welche durch saure Hydrolyse abspaltbar sind, können z. B. durch Behandeln mit einer anorganischen Säure, wie Chlorwasserstoffsäure, mit einer geeigneten Alkancarbonsäure (z. B. Essigsäure), welche, falls gewünscht, halogeniert sein kann (z. B. Trifluoressigsäure) oder mit einer Sulfonsäure, wie Toluol-4-sulfonsäure entfernt werden. Schutzgruppen, welche durch basische Hydrolyse abspaltbar sind, können z. B. durch Behandeln mit wässrigem Alkalimetallhydroxyd, wie wässrigem Natriumhydroxyd entfernt werden. Schutzgruppen, welche durch Hydrogenolyse abgespalten werden können, werden z. B. unter Verwendung von Wasserstoff in Gegenwart eines geeigneten Katalysators wie Palladium, etc. entfernt. Wenn $R^{20}$ eine geschützte Formylgruppe bedeutet, so kann diese in an sich bekannter Weise in die Formylgruppe übergeführt werden. Wenn z. B. $R^{20}$ eine Formylgruppe bedeutet, die in Form des Dimethyl- oder Diäthylacetals geschützt ist, kann die Formylgruppe durch Behandeln mit z. B. einer Alkansulfonsäure, wie Methansulfonsäure, einer aromatischen Sulfonsäure, wie Toluol-4-sulfonsäure, Trifluoressigsäure, eines starken Sulfonsäureharzes wie Amberlyst 15, Chlorwasserstoffsäure oder dergleichen freigesetzt werden.

Wenn das durch Reaktion einer Verbindung der allgemeinen Formel II mit einem reaktiven Derivat einer Säure der Formel III erhaltene Produkt zwei oder mehr Schutzgruppen enthält, sind diese Gruppen vorzugsweise von solcher Art, dass sie mit ein und derselben Methode abgespalten werden können. Die Schutzgruppen in den Ausgangsmaterialien werden deshalb vorzugsweise unter Berücksichtigung der obigen Tatsache ausgewählt. Andererseits können natürlich die Schutzgruppen auch solcher Art sein, dass sie nach verschiedenen Methoden abgespalten werden, in diesem Falle erfolgt die Abspaltung dieser Schutzgruppen schrittweise.

Die Behandlung einer Verbindung der Formel IV mit einem Alkalimetallhydroxyd gemäss Verfahrensaspekt (b) führt zur Oeffnung der N-Phthaloylgruppe unter Bildung einer 2-Carboxybenzoylgruppe. Zweckmässigerweise wird die Reaktion in einer wässrigen alkoholischen Lösung, wie einer wässrigen Methanollösung, durchgeführt. Natriumhydroxyd ist das bevorzugte Alkalimetallhydroxyd. Die Reaktion wird vorzugsweise bei Raumtemperatur durchgeführt.

Die allfälligen Schutzgruppen im erhaltenen Produkt können in derselben Weise entfernt werden, wie das im Zusammenhang mit der Entfernung der Schutzgruppen von Verbindungen angegeben wurde, die durch Reaktion einer Verbindung der Formel II mit einem reaktionsfähigen Derivat einer Säure der Formel III erhalten wurden.

Verbindungen der Formel I können durch Behandeln mit einer Base in pharmazeutisch annehmbare Salze übergeführt werden und Verbindungen der Formel I, welche eine freie Aminogruppe enthalten, können durch Behandeln mit einer Säure in ein pharmazeutisch annehmbares Säureadditionssalz übergeführt werden. Diese Ueberführungen erfolgen in an sich bekannter Weise.

Die Verbindungen der Formel II, welche als Ausgangsmaterialien gemäss dem Verfahren der vorliegenden Erfindung verwendet werden, sind neu und ebenfalls Gegenstand der vorliegenden Erfindung. Sie können hergestellt werden durch Ueberführung der geschützten Aminogruppe $R^6$ in einer Verbindung der allgemeinen Formel

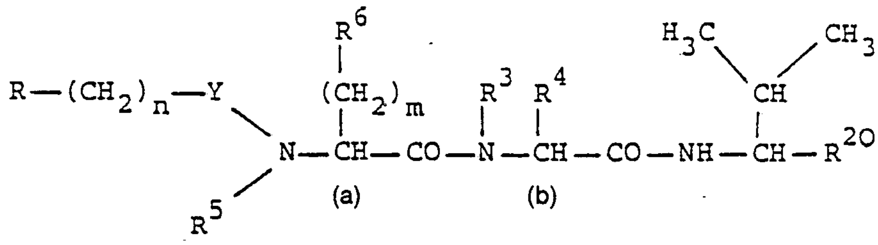

(V)

worin R, $R^3$, $R^4$, $R^5$, $R^{20}$, Y, m und n die frühere Bedeutung haben, $R^6$ eine geschützte Aminogruppe bedeutet, und die die mit (a) und (b) gekennzeichneten C-Atome die früher erwähnte Konfiguration aufweisen, in die freie Aminogruppe.

Die geschützte Aminogruppe $R^6$ in einer Verbindung der Formel V kann in ähnlicher Weise geschützt sein wie $R^5$ oder vorzugsweise eine Phthalimidogruppe sein. Wenn $R^6$ eine Phthalimidogruppe ist, so entspricht diese Verbindung der Formel V einer entsprechenden Verbindung der Formel IV.

Die Ueberführung der geschützten Aminogruppe $R^6$ in einer Verbindung der Formel V in die freie Aminogruppe kann in an sich bekannter Weise durchgeführt werden ; z. B. wie früher beschrieben oder, wenn die geschützte Aminogruppe eine Phthalimidogruppe ist, durch Behandeln mit Hydrazin in herkömmlicher Weise. Es ist klar, dass wenn mehr als eine Schutzgruppe in einer Verbindung der Formel V vorhanden sind, dass diese Gruppen so ausgewählt werden, dass lediglich die Schutzgruppe $R^6$ entfernt wird und dass die andern Schutzgruppen im Molekül nicht beeinträchtigt werden.

Die Verbindungen der Formel V sind neu und bilden einen weitern Aspekt der vorliegenden Erfindung. Sie können nach verschiedenen Methoden hergestellt werden.

Nach einer dieser Methoden wird eine Verbindung der Formel V in an sich bekannter Weise durch Reaktion einer Verbindung der allgemeinen Formel

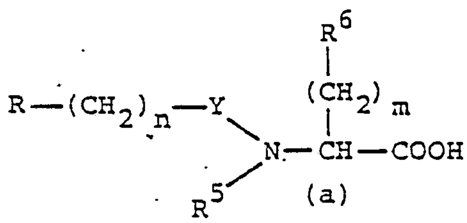

(VI)

worin R, $R^5$, $R^6$, Y, m und n die früher angegebene Bedeutung haben und das mit (a) gekennzeichnete C-Atom die früher angegebene Konfiguration hat, mit einer Verbindung der allgemeinen Formel

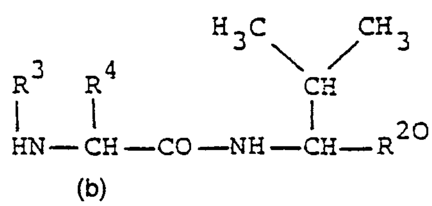

(VII)

worin $R^3$, $R^4$ und $R^{20}$ die früher angegebene Bedeutung haben und das mit (b) bezeichneten C-Atom die früher erwähnte Konfiguration hat, hergestellt.

Nach einer andern Methode erhält man Verbindungen der Formel V in an sich bekannter Weise durch Reaktion einer Verbindung der allgemeinen Formel

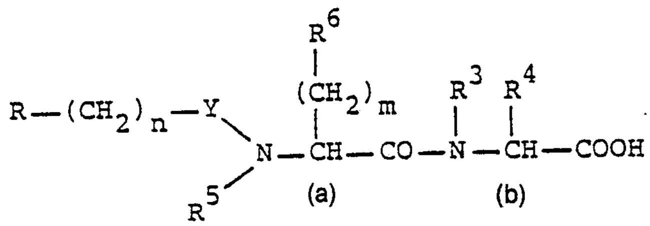

(VIII)

worin R, $R^3$, $R^4$, $R^5$, $R^6$, Y, m und n die früher angegebene Bedeutung haben und die mit (a) und (b) bezeichneten Kohlenstoffatomen die früher erwähnte Konfiguration haben, mit einer Verbindung der allgemeinen Formel

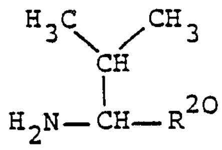

(IX)

worin $R^{20}$ die früher erwähnte Bedeutung hat.

Nach einer weiteren Methode können Verbindungen der Formel IV, worin Y eine Carbonyl- oder Sulfonylgruppe bedeutet, in an sich bekannter Weise durch Reaktion einer Verbindung der allgemeinen Formel

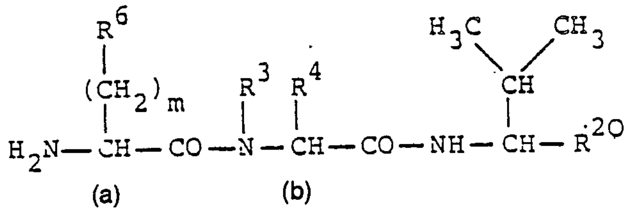

(X)

worin $R^3$, $R^4$, $R^6$, $R^{20}$ und m die früher angegebene Bedeutung haben und die (a) und (b) bezeichneten C-Atomen die früher erwähnte Konfiguration haben, mit einer Verbindung der Formel

$$R-(CH_2)_n-Y^1-X \qquad (XI)$$

worin R und n die früher angegebene Bedeutung haben, X Chlor oder Brom bedeutet und Y eine Carbonylgruppe, eine Sulfonylgruppe (Ausnahme, wenn R Adamantyl bedeutet) oder eine Sulfinylgruppe (wenn R Adamantyl bedeutet), bedeutet, und anschliessende Oxidation einer Verbindung, in welcher $Y^1$ eine Sulfinylgruppe bedeutet, zu einer entsprechenden Verbindung der Formel V, in der Y eine Sulfonylgruppe bedeutet, erhalten werden.

Nach einem weiteren Aspekt können Verbindungen der Formel V, worin Y eine Methylengruppe bedeutet, in an sich bekannter Weise durch reduktive Alkylierung einer Verbindung der Formel X mit einem Aldehyd der allgemeinen Formel

$$R-(CH_2)_n-CHO \qquad (XII)$$

worin R und n die früher angegebene Bedeutung haben, erhalten werden.

Verbindungen der Formel VI können erhalten werden, indem man eine geeignete Verbindung der allgemeinen Formel

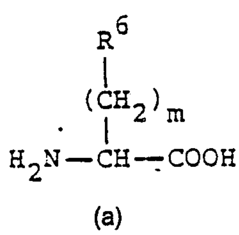

(XIII)

worin $R^6$ und m die früher erwähnte Bedeutung haben und das mit (a) bezeichneten C-Atom die früher erwähnte Konfiguration hat, mit einer Verbindung der allgemeinen Formel

$$R-(CH_2)_n-Y^2-X \qquad (XIV)$$

worin R, X und n die früher erwähnte Bedeutung haben und $Y^2$ die gleiche Bedeutung wie Y hat, aber eine Carbonyl-, Methylen- oder Sulfinylgruppe bedeutet, wenn R eine Adamantylgruppe bedeutet, zur Reaktion bringt und eine erhaltene Verbindung, worin $Y^2$ eine Sulfinylgruppe bedeutet zu einer entsprechenden Verbindung der Formel VI, worin Y eine Sulfonylgruppe bedeutet, oxidiert oder indem man eine Verbindung der Formel XIII mit einem Aldehyd der Formel XII reduktiv alkyliert und anschliessend die Schutzgruppe $R_5$ einführt.

Die Verbindungen der Formeln VII, IX, XI, XII, XIII und XIV sind entweder bekannt oder können in Analogie zur Herstellung der bekannten Verbindungen hergestellt werden.

Verbindungen der Formel VIII können z. B. durch Reaktion einer Verbindung der Formel VI mit einem L-Prolin- oder L-Alaninester (z. B. Methyl, Aethyl, tert. Butyl oder Benzylester) und anschliessende Abspaltung der Esterschutzgruppe erhalten werden.

Verbindungen der Formel X können z. B. durch Reaktion einer Verbindung der Formel VII mit einer entsprechenden Verbindung der Formel XIII, in welcher jedoch die Aminogruppe eine Schutzgruppe $R^5$

trägt und anschliessendes Abspalten der Schutzgruppe $R^5$ im Reaktionsprodukt erhalten werden.

Die reaktiven Derivate der Säuren der Formel III, welche als Ausgangsmaterialien verwendet werden, sind bekannte Verbindungen.

Die Verbindungen der Formel I und ihre pharmazeutisch annehmbaren Salze können zur Behandlung von degenerativen Erkrankungen, wie Emphysem und Arthritis, welche mit der Wirkung von Elastase-ähnlichen Enzymen begleitet sind, verwendet werden. Sie können ebenfalls zur Behandlung von entzündlichen Zuständen verwendet werden, bei denen Elastase-ähnliche Enzyme als Vermittler der Entzündung agieren.

Die pharmakologische Wirkung der Verbindungen der Formel I und ihre pharmazeutisch annehmbaren Salze wird anhand des nachfolgenden Tests gezeigt.

Test bezüglich der Inhibierung von menschlicher Granulozytenelastase

Dieser Test basiert auf der Methode von I. Pieth, B. Spiess und C. G. Wermuth in Bio. Chem. 11 (1974), 350.

Succinyl (Ala) 3-p-Nitroanilid (20 µl einer 125 mMol-Lösung in n-Methylpyrrolidon) wird in einem Puffer (3 ml, 0,2M Tris-HCl., pH 8,0) gelöst und auf 37 °C erwärmt. Menschliche Granulozytenelastase (4 µg in 20 µl Puffer) wird zugegeben, um die Reaktion zu starten, welche nach 1 Stunde bei 37 °C durch die Zugabe von Eisessig (100 µl) beendet wird. Freigesetztes p-Nitroanilin wird durch Messen der Extinktion der Lösung bei 410 nm mittels eines Spektrophotometers bestimmt. Die Verbindungen gemäss vorliegender Erfindung werden in 50 µl Methanol vor Zugabe der menschlichen Granulozytenelastase der gepufferten Substratlösung zugegeben, worauf die Substratfreisetzungsrate mit Lösungsmittelkontrollen verglichen wird. Es wird die Konzentration einer Verbindung gemäss vorliegender Erfindung bestimmt ($IC_{50}$), die benötigt wird, um eine 50 %ige Inhibierung zu bewirken.

Die Ergebnisse der Versuchs sind in der nachfolgenden Tabelle zusammengestellt :

Tabelle

| Verbindung gemäss vorliegender Erfindung | $IC_{50}$ |
|---|---|
| $N^{\alpha}$-(1-Adamantylsulphonyl)-$N^{\varepsilon}$-succinyl-L-lysyl-L-prolyl-L-valinal | $8 \times 10^{-8}$M |
| $N^{\alpha}$-(1-Adamantyläthyl)-$N^{\varepsilon}$-succinyl-L-lysyl-L-prolin-isobutylamid | $4{,}5 \times 10^{-6}$M |
| $N^{\alpha}$-(6,6-Dimethylbicyclo[3.3.1]heptyläthyl)-$N^{\varepsilon}$-(γ-glutamyl)-L-lysyl-L-prolin-isobutylamid | $1{,}6 \times 10^{-5}$M |
| $N^{\alpha}$-(1-Adamantylacetyl)-$N^{\varepsilon}$-succinyl-L-lysyl-L-prolin-isobutylamid | N/V |
| $N^{\alpha}$-(1-Adamantylsulphonyl)-$N^{\varepsilon}$-(2-carboxybenzoyl)-L-lysyl-L-prolylvalinal | $9 \times 10^{-8}$M |
| $N^{\alpha}$-(1-Adamantylsulphonyl)-$N^{\varepsilon}$-succinyl-L-lysyl-L-alanylvalinal | $1{,}2 \times 10^{-7}$M |
| $N^{\alpha}$-(1-Adamantylsulphonyl)-$N^{\varepsilon}$-(4-carboxybenzoyl)-L-lysyl-L-prolyl-L-valinal | $6 \times 10^{-8}$M |
| $N^{\alpha}$-(1-Adamantylsulphonyl)-$N^{\varepsilon}$-adipoyl-L-lysyl-L-prolylvalinal | $8 \times 10^{-8}$M |
| $N^{\alpha}$-(1-Adamantylsulphonyl)-$N^{\varepsilon}$-(4-carboxybenzolyl)-L-lysyl-L-alanyl-L-valinal | $5 \times 10^{-8}$M |

N/V = Nicht verfügbar

Die Verbindungen der Formel I und ihre pharmazeutisch annehmbaren Salze können als Medikamente in Form von pharmazeutischen Präparaten verwendet werden, welche diese mit einem geeigneten pharmazeutischen Trägermaterial enthalten. Dieses Trägermaterial kann ein organisches oder anorganisches Trägermaterial sein, welches für enterale oder parenterale Administration geeignet ist, wie Wasser, Lactose, Stärke, Magnesiumstearat, Talk, Gummi arabicum, Gelatine, Polyalkylenglykol, Vaselin, etc. Die pharmazeutischen Präparate können als Festformen (z. B. als Tabletten, Pulver, Dragées, Suppositorien, Kapseln, etc.) oder als flüssige Formen (z. B. als Lösungen, Emulsionen, Suspensionen, etc.) hergestellt werden. Die pharmazeutischen Präparate sind jedoch besonders bevorzugt in der Form eines Aerosols, welches zweckmässigerweise zusätzlich zum Treibgas ein Lösungsmittel wie Aethanol enthält.

Falls nötig können die pharmazeutischen Präparate gemäss vorliegender Erfindung herkömmlichen pharmazeutischen Techniken unterzogen werden, wie Sterilisierung und dergleichen und sie können auch herkömmliche pharmazeutische Adjuvantien, wie Konservierungsmittel, Stabilisierungsmittel, Netzmittel, Salze zur Veränderung des osmotischen Druck, etc. enthalten. Die pharmazeutischen Präparate gemäss vorliegender Erfindung können auch andere therapeutisch wertvolle Substanzen enthalten.

Die pharmazeutischen Präparate gemäss vorliegender Erfindung können durch Mischen einer Verbindung der Formel I oder eines pharmazeutisch annehmbaren Salzes davon mit einem kompatiblen pharmazeutischen Trägermaterial und Einbringen dieses Gemisches in die gewünschte pharmazeutische Darreichungsform erhalten werden.

Die Verbindungen der Formel I und ihre pharmazeutisch annehmbaren Säureadditionssalze können Erwachsenen in einem Dosisbereich von ungefähr 5 mg bis 30 mg, vorzugsweise ungefähr 10 mg bis 15 mg pro Tag verabreicht werden. Selbstverständlich ist dieser Dosisbereich nur als Beispiel angegeben und kann aufwärts oder abwärts variieren, was von Faktoren, wie der Wirkungsstärke der bestimmten zu verabreichenden Verbindung, des bestimmten zu behandelnden Zustandes und den individuellen Gegebenheiten des Patienten, wie sie durch den Arzt bestimmt werden, abhängt.

Die folgenden Beispiele erläutern die vorliegende Erfindung. Die Struktur der Produkte wurde durch NMR-Spektroskopie und Massenspektroskopie bestätigt.

Beispiel 1

(A) 4,55 g (0,006 5 Mol) $N^{\alpha}$-(1-Adamantylsulfonyl)-$N^{\varepsilon}$-phthaloyl-L-lysyl-L-prolyl-L-valinal-dimethylacetal in 30 ml Aethanol und 0,94 ml Hydrazinhydrat werden während 16 Stunden bei Raumtemperatur gerührt. Die Lösung wird filtriert und das Filtrat wird eingedampft. Durch zweifaches Eindampfen mit je 20 ml Aethanol werden Spuren von Hydrazinhydrat entfernt. Der Rückstand wird dann in Aethanol gelöst, worauf die Lösung nach Zugabe von 0,9 ml Essigsäure während 20 Minuten gerührt wird. Das Lösungsmittel wird durch Eindampfen entfernt. Durch dreimaliges Eindampfen mit je 20 ml Aethanol werden Spuren von Essigsäure entfernt. Der Rückstand wird in 20 ml Chloroform aufgenommen, worauf die Lösung bei 0 °C stehengelassen wird. Nach Filtrieren und Eindampfen des Filtrates erhält man einen Schaum. Dieser Schaum wird in 15 ml Tetrahydrofuran aufgenommen, worauf die Lösung mit 2,76 ml (0,019 5 Mol) Triäthylamin und 1,3 g (0,013 Mol) Bernsteinsäureanhydrid behandelt und dann während 16 Stunden bei Raumtemperatur stehengelassen wird. Das Lösungsmittel wird durch Abdampfen entfernt und der Rückstand wird in Essigester/Natriumbicarbonatlösung aufgenommen. Die wässrige Phase wird abgetrennt, mit Essigester extrahiert und mit verdünnter Salzsäure auf pH 3-4 angesäuert. Das Produkt wird mit drei 20 ml-Portionen Chloroform extrahiert. Die Chloroformextrakte werden mit Wasser gewaschen, über Magnesiumsulfat getrocknet und eingedampft und liefern 3,9 g (89 %) $N^{\alpha}$-(1-Adamantylsulfonyl)-$N^{\varepsilon}$-succinyl-L-lysyl-L-prolyl-L-valinal-dimethylacetal als Schaum ; Rf-Wert [Chloroform/Methanol/Essigsäure/Wasser (120 : 15 : 3 : 2)] : 0,52.

(B) 0,46 g (0,69 mMol) $N^{\alpha}$-(1-Adamantylsulfonyl)-$N^{\varepsilon}$-succinyl-L-lysyl-L-prolyl-L-valinal-dimethylacetal in 25 ml Aceton werden während 16 Stunden bei Raumtemperatur mit 0,14 g Toluol-4-sulfonsäure behandelt. Das Lösungsmittel wird durch Eindampfen entfernt und der Rückstand wird mit 7 ml Chloroform aufgenommen, worauf die Lösung mit Wasser gewaschen, über Magnesiumsulfat getrocknet und eingedampft wird. Man erhält einen Schaum. Durch Chromatographie an 60 g Silicagel unter Verwendung von Chloroform/Methanol/Essigsäure/Wasser (120 : 15 : 3 : 2) für die Eluierung und anschliessende Lyophilisierung aus Essigsäure/Wasser (1 : 4) erhält man 0,13 g (30 %) $N^{\alpha}$-(1-Adamantylsulfonyl)-$N^{\varepsilon}$-succinyl-L-lysyl-L-prolyl-L-valinal. Rf-Wert [Chloroform/Methanol/Essigsäure/Wasser (120 : 15 : 3 : 2)] : 0,32 ; $[\alpha]_D^{25} = -41.8°$ (c = 0,25 in 50 % Essigsäure).

Analyse für $C_{30}H_{48}N_4O_8S$ (624,80) :

Berechnet : C 58,29 H 7,89 N 8,77 %

Gefunden : C 56,20 H 7,62 N 8,54 %

C 56,23 H 7,75 N 8,52 % (mit 0,5 Mol Wasser und 0,4 Mol Essigsäure).

Das $N^{\alpha}$-(1-Adamantylsulphonyl)-$N^{\varepsilon}$-phthaloyl-L-lysyl-L-prolyl-L-valinyldimethylacetal, welches in Paragraph (A) als Ausgangsmaterial verwendet wird, wird wie folgt hergestellt :

(a) 22,8 g (0,097 Mol) N-Benzyloxycarbonyl-L-valinal werden in 55 ml trockenem Methanol gelöst. Die

Lösung wird mit 10,8 g (0,102 Mol) Trimethylorthoformiat und 0,3 g Toluol-4-sulfonsäure behandelt, worauf das Gemisch während 16 Stunden bei Raumtemperatur stehengelassen wird. Die Lösungsmittel werden durch Eindampfen entfernt und der Rückstand wird in 150 ml Essigester gelöst, worauf die Lösung mit 5 %-iger Natriumbicarbonatlösung und dann mit Sole gewaschen wird. Nach Trocknen über Magnesiumsulfat und Filtration wird das Lösungsmittel durch Eindampfen entfernt. Man erhält 24,5 g (90 %) N-Benzyloxycarbonyl-L-valinal-dimethylacetat als ein Oel ; Rf-Wert [Hexan-Essigester (2 : 1)] : 0,69.

(b) 24,5 g (0,087 Mol) N-Benzyloxycarbonyl-L-valinal-dimethylacetal werden in 200 ml Methanol gelöst und die Lösung wird während 2 Stunden in Gegenwart von 0,5 g 5 %-iger Palladium/Kohle hydriert. Nach Entfernung des Katalysators durch Filtrieren wird das Lösungsmittel durch Eindampfen entfernt und der Rückstand in 250 ml Dichlormethan gelöst. Die Lösung wird mit Sole gewaschen und über Magnesiumsulfat getrocknet. Die Lösung wird dann auf — 20 °C abgekühlt, worauf man 21,8 g (0,087 Mol) N-Benzyloxycarbonyl-L-prolin und anschliessend 19,8 g (0,096 Mol) N,N'-Dicyclohexylcarbodiimid zugibt. Die Lösung wird während 2 Stunden bei — 20 °C gerührt und dann während 64 Stunden bei 4 °C stehengelassen. Der gebildete N,N'-Dicyclohexylharnstoff wird durch Filtration entfernt und das Filtrat wird eingedampft. Der Rückstand wird in Essigester aufgenommen und die Lösung wird mit Natriumbicarbonat und Sole gewaschen und dann über Magnesiumsulfat getrocknet und zu einem Oel eingedampft. Das Produkt wird durch Chromatographie an 250 g Silicagel unter Verwendung von Essigester/Hexan (1 : 1) zur Eluierung gereinigt. Durch Eindampfen der Lösung erhält man 21,9 g (67 %) N-Benzyloxycarbonyl-L-prolyl-L-valinal-dimethylacetal ; Rf-Wert (Essigester) : 0,63.

(c) 6,05 g (0,016 Mol) N-Benzyloxycarbonyl-L-prolyl-L-valinal-dimethylacetal werden in 60 ml Methanol gelöst und die Lösung wird während 4 Stunden in Gegenwart von 0,25 g von 5 % Palladium/Kohle hydriert. Nach Entfernen des Katalysators durch Filtrieren wird das Lösungsmittel durch Abdampfen entfernt, worauf durch zweimaliges Eindampfen mit je 20 ml Toluol Spuren von Methanol entfernt werden. Eine Lösung des Rückstandes in 30 ml Dichlormethan wird auf 20 °C abgekühlt, worauf man 6,9 g (0,014 6 Mol) $N^{\alpha}$-(1-Adamantylsulfonyl)-$N^{\varepsilon}$-phthaloyl-L-lysin gefolgt von 3,36 g (0,016 Mol) N,N'-Dicyclohexylcarbodiimid zugibt. Das Gemisch wird während 2 Stunden bei — 20 °C gerührt und dann während 16 Stunden bei 4 °C stehengelassen. Der ausgefällte N,N'-Dicyclohexylharnstoff wird durch Filtrieren entfernt und das Filtrat wird mit Natriumbicarbonatlösung und Sole gewaschen, über Magnesiumsulfat getrocknet und zu einem Oel eingedampft. Das Produkt wird durch Chromatographie an 100 g Silicagel unter Verwendung von Hexan/Essigester (4 : 1) und dann Hexan/Essigester (1 : 3) als Eluiermittel gereinigt. Durch Eindampfen des Lösungsmittels erhält man 5,5 g (54 %) $N^{\alpha}$-(1-Adamantylsulfonyl)-$N^{\varepsilon}$-phthaloyl-L-lysyl-L-propyl-L-valinal-dimethylacetal ; Rf-Wert (Essigester) : 0,57.

Analyse für $C_{36}H_{52}N_4O_8S$ (700,90) :
Berechnet : C 61,69 H 7,48 N 7,99 %
Gefunden : C 61,48 H 7,35 N 7,91 %.

Das $N^{\alpha}$-(1-Adamantylsulfonyl)-$N^{\varepsilon}$-phthaloyl-L-lysin welches in Paragraph (c) verwendet wird, wird wie folgt hergestellt :

(a) 27,0 g (0,098 Mol) $N^{\varepsilon}$-Phthaloyl-L-lysin werden in 500 ml Dimethylformamid suspendiert. Nach Zugabe von 29,7 ml (0,235 Mol) N-Aethylmorpholin und 32 g (0,147 Mol) 1-Adamantansulphinylchlorid wird das Gemisch während 16 Stunden bei Raumtemperatur gerührt. Das Lösungsmittel wird durch Eindampfen entfernt und der Rückstand wird in Essigester/5 %-ige Natriumbicarbonatlösung gelöst. Die wässrige Schicht wird mit Essigester extrahiert und dann mit konzentrierter Salzsäure auf pH 2 angesäuert. Das ölige Produkt wird in Essigester extrahiert, worauf einiges Produkt auskristallisiert. Der Feststoff (hauptsächlich ein Diastereomeres) wird abfiltriert, mit Wasser und Diäthyläther gewaschen und im Vakuum getrocknet. Die Essigesterlösung wird mit Wasser und Sole gewaschen, über Magnesiumsulfat getrocknet und zur Trockene eingedampft. Durch 16-stündiges Verreiben mit Diäthyläther bei 0 °C erhält man einen Feststoff, welcher abfiltriert, mit Diäthyläther gewaschen und im Vakuum getrocknet wird. Dieser Feststoff wird mit dem früher erhaltenen Feststoff zusammengelegt, wobei man 27,2 g (61 %) $N^{\alpha}$-(1-Adamantylsulphinyl)-$N^{\varepsilon}$-phthaloyl-L-lysin als Diastereoisomerengemisch erhält ; Rf-Wert [Methanol/Chloroform (1 : 9)] : 0,53 und 0,27.

(b) 27,2 g (0,059 Mol) N-(1-Adamantylsulphinyl)-$N^{\varepsilon}$-phthaloyl-L-lysin in 200 ml Eisessig wird mit 27 ml 30 %-igem (Gewicht/Volumen) Wasserstoffperoxidlösung während 2 Stunden bei 65 °C behandelt. Die Lösung wird auf Raumtemperatur abgekühlt und auf 1,5 l Wasser gegossen, worauf das Produkt in 200 ml Essigester extrahiert wird. Die organische Lösung wird dreimal mit je 100 ml Wasser und Sole gewaschen, über Magnesiumsulfat getrocknet und zu einem Oel eingedampft. Durch zweifaches Eindampfen mit je 50 ml Toluol werden die letzten Spuren der Essigsäure entfernt. Durch Trocknen im Hochvakuum erhält man 21,7 g (78 %) $N^{\alpha}$-(1-Adamantylsulphonyl)-$N^{\varepsilon}$-phthaloyl-L-lysin als weissen Schaum ; Rf-Wert [Methanol/Chloroform (1,19)] : 0,57 ; $[\alpha]_D^{20}$ = — 14,8° (c = 1 % in Dimethylformamid).

Analyse für $C_{24}H_{30}N_2O_6S$ (474,6) :

Berechnet : C 60,74 H 6,37 N 5,90 %
Gefunden : C 60,19 H 6,29 N 5,99 ; Wasser : 1,42 %
Wasserfrei : C 61,05 H 6,21 N 6,07 %.

Beispiel 2

(A) 2,4 g (0,003 4 Mol) $N^{\alpha}$-(1-Adamantyläthyl)-$N^{\alpha}$-benzyloxycarbonyl-$N^{\varepsilon}$-tertbutoxycarbonyl-L-lysyl-L-prolin-isobutylamid werden während einer halben Stunde bei Raumtemperatur mit 10 ml 4M Salzsäure in Essigester behandelt. Der durch Zugabe von Diäthyläther erhaltene weisse Feststoff wird mit Diäthyläther gewaschen und am Rotationsverdampfer eingedampft. Eine Lösung dieses Feststoffes in Dimethylformamid wird mit N-Aethylmorpholin auf pH 8 gestellt, mit 0,68 g (0,006 8 Mol) Bernsteinsäureanhydrid versetzt, worauf der pH der entstandenen Lösung während 2 Stunden durch die tropfenweise Zugabe von N-Aethylmorpholin bei pH 8 gehalten wird. Das Lösungsmittel wird eingedampft und der Rückstand wird in Essigester/wässriger Salzsäure (pH 3) gelöst. Die organische Phase wird mit Wasser gewaschen, das Produkt mit 2 × 50 ml 5 %-iger Natriumbicarbonatlösung extrahiert. Die Bicarbonatlösungen werden vereinigt und mit 2M Salzsäure auf pH 3 gestellt. Das Produkt wird mit 2 × 50 ml Essigester extrahiert. Die Essigesterextrakte werden kombiniert, mit Wasser gewaschen und über Magnesiumsulfat getrocknet. Durch Eindampfen des Lösungsmittels erhält man 2,0 g (85 %) $N^{\alpha}$-(1-Adamantyläthyl)-$N^{\alpha}$-benzyloxycarbonyl-$N^{\varepsilon}$-succinyl-L-lysyl-L-prolin-isobutylamid in Form eines Schaums.

Analyse für $C_{39}H_{58}N_4O_7$ (694,92) :
Berechnet : C 67,41 H 8,41 N 8,06 %
Gefunden : C 67,06 H 8,24 N 7,74 %.

(B) 2,0 g (0,002 9 Mol) $N^{\alpha}$-(1-Adamantyläthyl)-$N^{\alpha}$-benzyloxycarbonyl-$N^{\varepsilon}$-succinyl-L-lysyl-L-prolin-isobutylamid werden in 50 ml Methanol gelöst und die Lösung wird während 3 Stunden in Gegenwart von 0,2 g 5 % Palladium/Kohle hydriert. Der Katalysator wird durch Filtrieren entfernt und das Filtrat wird eingedampft. Man erhält 1,6 g (98 %) $N^{\alpha}$-(1-Adamantyläthyl)-$N^{\varepsilon}$-succinyl-L-lysyl-L-prolin-isobutylamid als einen Schaum ; $[\alpha]_D^{20}$ = —47,2° (c = 1 in Methanol).

Analyse für $C_{31}H_{52}N_4O_5$ (560,78) :
Berechnet : C 66,40 H 9,35 N 9,99 %
Gefunden : C 66,52 H 9,09 N 9,80 %.

Das $N^{\alpha}$-(1-Adamantyläthyl)-$N^{\alpha}$-benzyloxycarbonyl-$N^{\varepsilon}$-tert.butoxycarbonyl-L-lysyl-L-prolin-isobutylamid, welches als Ausgangsmaterial in Paragraph (A) verwendet wird, wird wie folgt hergestellt :

(a) 35,6 g (0,143 Mol) N-Benzyloxycarbonyl-L-prolin werden in 200 ml Tetrahydrofuran gelöst und die Lösung wird auf —20 °C gekühlt. 18,1 ml (0,134 Mol) N-Aethylmorpholin und 08,6 ml (0,143 Mol) Chlorameisensäureisobutylester werden zugegeben, worauf das Gemisch während 4 Minuten bei —20 °C gerührt wird. Nach Zugabe von 14,2 ml (0,143 Mol) Isobutylamin wird das Reaktionsgemisch während 2 Stunden bei 0 °C und dann während 16 Stunden bei Raumtemperatur gerührt. Das Lösungsmittel wird durch Eindampfen entfernt und der Rückstand wird in Essigester gelöst. Die erhaltene Lösung wird mit 5 %-iger Zitronensäurelösung, Wasser, 5 %-iger Natriumbicarbonatlösung und Sole gewaschen, über Magnesiumsulfat getrocknet und eingedampft. Durch Verreiben des als Rückstand erhaltenen Oeles mit Petroläther (40 °C bis 60 °C) erhält man 31,3 g (72 %) N-Benzyloxycarbonyl-L-prolylisobutylamid vom Schmelzpunkt 84-85 °C ; $[\alpha]_D^{20}$ = —53,2° (c = 1 in Methanol).

Analyse für $C_{17}H_{24}N_2O_3$ (304,39) :
Berechnet : C 67,08 H 7,95 N 9,20 %
Gefunden : C 66,92 H 7,92 N 9,07 %.

(b) 31,1 g (0,102 Mol) N-Benzyloxycarbonyl-L-prolin-isobutylamid werden in 300 ml Dimethylformamid gelöst und die Lösung wird während 16 Stunden in Gegenwart von 3 g 5 % Palladium/Kohle während 16 Stunden hydriert. Der Katalysator wird durch Filtrieren entfernt und das Filtrat wird auf 0 °C gekühlt. 49,1 g (0,101 Mol) $N^{\alpha}$-Benzyloxycarbonyl-$N^{\alpha}$-tert.-butoxycarbonyl-L-lysin-N-hydroxysuccinimidester, welcher in 250 ml Dimethylformamid gelöst ist, wird zur Lösung gegeben und das Reaktionsgemisch wird während einer Stunde bei 0 °C und während 16 Stunden bei Zimmertemperatur gerührt. Das Lösungsmittel wird durch Eindampfen entfernt und der Rückstand wird in Essigester/5 %-iger Zitronensäurelösung gelöst. Die organische Phase wird mit Wasser, 5 %-iger Natriumbicarbonatlösung und Sole gewaschen, über Magnesiumsulfat getrocknet und eingedampft. Durch Kristallisation des Rückstandes aus Diäthyläther/Petroläther erhält man 36,4 g (57 %) $N^{\alpha}$-Benzyloxycarbonyl-$N^{\varepsilon}$-tert.-butoxycarbonyl-L-lysyl-L-prolin-isobutylamid vom Schmelzpunkt 105-107 °C.

(c) Eine Lösung von 6.3 g (0,012 Mol) von $N^{\alpha}$-Benzyloxycarbonyl-$N^{\varepsilon}$-tert.butoxycarbonyl-L-lysyl-L-prolin-isobutylamid in 100 ml Methanol wird während 3 Stunden in Gegenwart von 0,6 g 5 % Palladium/Kohle hydriert. Nach Zugabe einer Lösung von 2,2 g Adamantan-Adetaldehyd in 5 ml Methanol wird

die Hydrierung während 16 Stunden fortgesetzt. Der Katalysator wird durch Filtrieren entfernt und das Filtrat wird eingedampft. Eine Lösung des Rückstandes in 70 ml Diäthyläther wird mit 60 ml Wasser welche 2,5 g (0,025 Mol) Natriumbicarbonat enthalten, behandelt. Nach Zugabe von 1,85 ml (0,023 Mol) Chlorameisensäurebenzylester wird das Reaktionsgemisch während einer Stunde kräftig gerührt. Die organische Phase wird abgetrennt mit 5 %-iger zitronensaurer Lösung, Wasser, 5 % Natriumbicarbonatlösung und Sole gewaschen, über Magnesiumsulfat getrocknet und zu einem Schaum eingedampft. Durch Chromatographie an 150 g Silicagel unter Verwendung von n-Hexan/Essigester (2 : 1) als Eluiermittel erhält man 4,4 g (53 %) $N^{\alpha}$-(1-Adamantyläthyl)-$N^{\alpha}$-benzyloxycarbonyl)-$N^{\varepsilon}$-tert.butoxycarbonyl-L-lysyl-L-prolin-isobutylamid als Schaum ; Rf-Wert [n-Hexan/-Essigester (2 : 1)] : 0,49.

## Beispiel 3

0,9 g (0,001 5 Mol) $N^{\alpha}$-(1-Adamantanacetyl)-$N^{\varepsilon}$-benzyloxycarbonyl-L-lysyl-L-prolin-isobutylamid werden in 3 ml Essigsäure gelöst, worauf 3 ml einer 45 %igen Bromwasserstofflösung zugegeben werden. Nach 1 Stunde wird trockener Diäthyläther zugegeben, worauf der ausgefallene Feststoff abfiltriert, mit Diäthyläther gewaschen und getrocknet wird. Der Feststoff wird in 10 ml Dimethylformamid aufgenommen und die Lösung wird mit 0,41 ml (0,003 3 Mol) N-Aethylmorpholin und 0,3 g (0,003 Mol) Bernsteinsäureanhydrid während 16 Stunden bei Raumtemperatur behandelt. Das Lösungsmittel wird eingedampft und der Rückstand wird in Essigester/verdünnter Salzsäure (pH 3) gelöst. Die organische Lösung wird mit zweimal je 15 ml 5 %iger Natriumbicarbonatlösung extrahiert. Nach Ansäuern auf pH 3 wird mit Essigester extrahiert, mit Wasser gewaschen, über Magnesiumsulfat getrocknet und eingedampft. Man erhält ein Rohprodukt. Dieses wird an 10 g Silicagel unter Verwendung von Essigester enthaltend 3 % Methanol als Eluiermittel chromatographiert und gibt 0,37 g (43 %) $N^{\alpha}$-(1-Adamantanacetyl)-$N^{\varepsilon}$-succinyl-L-lysyl-L-prolin-isobutylamid als ein Oel ; Rf-Wert [Methanol/Chloroform (3 : 97)] : 0,4.

Analyse für $C_{31}H_{49}N_4O_6$ (573,76) :

Das $N^{\alpha}$-(1-Adamantanacetyl)-$N^{\varepsilon}$-benzyloxycarbonyl-L-lysyl-L-prolin-isobutylamid, welches im vorerwähnten Absatz als Ausgangsmaterial verwendet wird, erhält man wie folgt :

(a) 6,1 g (0,02 Mol) N-Benzyloxycarbonyl-L-prolin-isobutylamid werden während 1 Stunde bei Raumtemperatur mit 15 ml 45 %igem Bromwasserstoff in Essigsäure gerührt. Das nach Zugage von trockenem Diäthyläther erhaltene Oel wird durch Dekantieren mit Diäthyläther gewaschen und am Rotationsverdampfer getrocknet. Der Rückstand wird in 50 ml Tetrahydrofuran gelöst und die Lösung mit N-Aethylmorpholin neutralisiert. Diese Lösung wird zu einer Lösung von gemischtem Anhydrid gegeben, welch letzteres wie folgt erhalten wurde : 7,6 g (0,02 Mol) $N^{\alpha}$-tert.Butoxycarbonyl-$N^{\varepsilon}$-benzyloxycarbonyl-L-lysin in 100 ml Tetrahydrofuran werden mit 2,54 ml (0,02 Mol) N-Aethylmorpholin und 2,62 ml (0,02 Mol) Chlorameisensäurebutylester während 4 Minuten bei — 20 °C gerührt.

Die Mischung wird während 2 Stunden bei 0 °C gerührt, das Lösungsmittel durch Eindampfen entfert und der Rückstand wird in Essigester/Wasser gelöst. Die organische Lösung wird mit 5 %iger Zitronensäurelösung, Wasser, 5 %iger Natriumbicarbonatlösung und Sole gewaschen und dann über Magnesiumsulfat getrocknet. Durch Eindampfen des Lösungsmittels erhält man 9,1 g (85 %) $N^{\alpha}$-tert.Butoxycarbonyl-$N^{\varepsilon}$-benzyloxycarbonyl-L-lysyl-L-prolin-isobutylamid als Oel ; Rf-Wert (Methanol/Chloroform [1 : 9]) : 0,75.

(b) 2,72 g (0,005 Mol) $N^{\alpha}$-tert.Butoxycarbonyl-$N^{\varepsilon}$-benzyloxycarbonyl-L-lysyl-L-prolin-isobutylamid werden während einer 3/4 Stunde bei Raumtemperatur mit einer Lösung von 15 ml von 2,5M Salzsäure in Essigester behandelt. Das Lösungsmittel wird durch Eindampfen entfernt und der Rückstand wird mit Diäthyläther gewaschen. Der Feststoff wird in 15 ml Dimethylformamid gelöst und die Lösung wird auf 0 °C abgekühlt. 1,27 ml (0,01 Mol) N-Aethylmorpholin und 1,3 g (0,006 Mol) 1-Adamantanacetylchlorid werden zugegeben, worauf das Gemisch während 3 Stunden bei Raumtemperatur gerührt wird. Das Lösungsmittel wird durch Eindampfen entfernt und der Rückstand wird in Essigester/Wasser gelöst. Die organische Lösung wird mit einer 5 %igen zitronensauren Lösung, Wasser, einer 5 %igen Natriumbicarbonatlösung und mit Sole gewaschen, über Magnesiumsulfat getrocknet und eingedampft. Durch Chromatographie an 60 g Silicagel unter Verwendung von Essigester als Eluiermittel erhalt man 0,9 g (29 %) $N^{\alpha}$-(1-Adamantanacetyl)-$N^{\varepsilon}$-benzyloxycarbonyl-L-lysyl-L-prolin-isobutylamid als Oel.

Analyse für $C_{35}H_{52}N_4O_5$ (608,83) :

| | | | |
|---|---|---|---|
| Berechnet : | C 69,05 | H 8,61 | N 9,20 % |
| Gefunden : | C 68,94 | H 8,45 | N 9,14 %. |

## Beispiel 4

(A) 14,8 g (0,021 Mol) $N^{\alpha}$-(1-Adamantylsulfonyl)-$N^{\varepsilon}$-phthaloyl-L-lysyl-L-prolyl-L-valinal-dimethylacetal in 75 ml Methanol und eine Lösung von 0,94 g (0,023 5 Mol) Natriumhydroxyd in 75 ml Wasser werden bei Raumtemperatur während einer halben Stunde gerührt. Das Methanol wird durch Eindampfen entfernt und die Lösung wird mit Wasser verdünnt und zweimal mit 50 ml Essigester extrahiert. Die

wässrige Lösung wird mit verdünnter Salzsäure auf pH 2-3 angesäuert und das entstandene ölige Produkt wird mit zweimal 75 ml Essigester extrahiert. Die Lösung wird mit Wasser gewaschen, über Magnesiumsulfat getrocknet und liefert nach dem Eindampfen einen Schaum. Der Schaum wird in Dichlormethan aufgenommen, mit Diäthyläther behandelt, gekühlt und liefert 14,4 g (95 %) $N^{\alpha}$-(1-Adamantylsulfonyl)-$N^{\varepsilon}$-(2-carboxybenzoyl)-L-lysyl-L-prolyl-L-valinal-dimethylacetal in Form eines amorphen Feststoffes.

(B) 0,5 g (0,000 7 Mol) $N^{\alpha}$-(1-Adamantylsulfonyl)-$N^{\varepsilon}$-(2-carboxybenzoyl)-L-lysyl-L-prolyl-L-valinal-dimethylacetal in 15 ml trockenem Aceton wird in 0,5 g Amberlyst 15 Harz behandelt, worauf das Gemisch während 16 Stunden bei Raumtemperatur gerührt wird. Das Harz wird abfiltriert, mit 10 ml Aceton gewaschen und die vereinigten Filtrate und Waschflüssigkeiten werden eingedampft. Das erhaltene Oel wird an 25 g Silicagel unter Verwendung von Dichlormethan/Methanol/Essigsäure (190 : 9 : 2) als Eluiermittel chromatographiert. Nach Eindampfen der Eluate wird der Rückstand mit Diäthyläther verrieben und liefert 0,12 g (26 %) $N^{\alpha}$-(1-Adamantylsulfonyl)-$N^{\varepsilon}$-(2-carboxybenzoyl)-L-lysyl-L-prolin-valinal in Form eines amorphen hygroskopischen Pulvers. Das Produkt ist eine Mischung von zwei Epimeren.

Analyse für $C_{34}H_{48}N_4O_8S$ (672,84 :
Berechnet : C 60,69 H 7,19 N 8,33 %
Gefunden : C 60,35 H 7,37 N 8,03 %
C 60,37 H 7,21 N 8,28 % (mit 0,2 Mol Wasser).

Beispiel 5

(A) 10 g (0,014 8 Mol) $N^{\alpha}$-(1-Adamantylsulfonyl)-$N^{\varepsilon}$-phthaloyl-L-lysyl-L-alanyl-L-valinal-dimethylacetal wird in analoger Weise wie in Beispiel 1(A) erst mit Hydrazinhydrat und dann mit Bernsteinsäureanhydrid/Triäthylamin behandelt. Das Produkt wird durch Chromatographie an 150 g Silicagel unter Verwendung von Chloroform/Methanol/Essigsäure/Wasser (120 : 15 : 3 : 2) als Eluiermittel gereinigt. Nach Eindampfen des Eluates erhält man 4,56 g (48 %) $N^{\alpha}$-(1-Adamantylsulfonyl)-$N^{\varepsilon}$-succinyl-L-lysyl-L-alanyl-L-valinal-dimethylacetal in Form eines Schaums.

(B) 0,5 g (0,78 mMol) $N^{\alpha}$-(1-Adamantylsulfonyl)-$N^{\varepsilon}$-succinyl-L-lysyl-L-alanyl-L-valinal-dimethylacetal in 10 ml Dimethylformamid und 50 ml trockenem Aceton wird während 28 Stunden bei Raumtemperatur mit 5 g Amberlyst 15 Harz behandelt. Das Gemisch wird filtriert und das Harz wird mit Dimethylformamid gewaschen. Das Filtrat wird eingedampft und liefert ein Oel. Durch Chromatographie an 80 g Silicagel unter Verwendung von Chloroform/Methanol/Essigsäure/Wasser (120 : 15 : 3 : 2) als Eluiermittel gefolgt von einer Lyophilisation aus wässriger Essigsäure erhält man 0,3 g (64 %) $N^{\alpha}$-(1-Adamantylsulfonyl)-$N^{\varepsilon}$-succinyl-L-lysyl-L-alanyl-valinal als ein Gemisch von Epimeren.

Analyse für $C_{28}H_{46}N_4O_8S$ (598,76)
Berechnet : C 56,17 H 7,74 N 9,36 %
Gefunden : C 54,26 H 7,74 N 9,02 %
C 54,37 H 7,85 N 9,06 % (mit 1,1 Mol Wasser).

Das $N^{\alpha}$-(1-Adamantylsulfonyl)-$N^{\varepsilon}$-phthaloyl-L-lysyl-L-alanyl-L-valinal-dimethylacetal, welches als Ausgangsmaterial verwendet wird erhält man wie folgt :

7,0 g (0,02 Mol) N-Benzyloxycarbonyl-L-alanyl-L-valinal-dimethylacetal [hergestellt in ähnlicher Weise wie in Beispiel 1(b) beschrieben] werden in 70 ml Methanol gelöst und die Lösung wird während 4 Stunden in Gegenwart von 0,7 g von 5 % Palladium/Kohle hydriert. Nach Entfernung des Katalysators durch Filtration wird das Lösungsmittel durch Eindampfen entfernt und Spuren von Methanol werden vom Rückstand durch zweifaches Eindampfen mit je 20 ml Toluol entfernt. Eine Lösung des Rückstandes in 40 ml Dichlormethan wird auf — 20 °C gekühlt, worauf 8,53 g (0,018 Mol) $N^{\alpha}$-(1-Adamantylsulfonyl)-N-phthaloyl-L-lysin in 80 ml Dichlormethan gefolgt von 4,45 g (0,021 6 Mol) N,N'-Dicyclohexylcarbodiimid zugegeben werden. Die Mischung wird während 2 Stunden bei — 20 °C gerührt und dann während 16 Stunden bei 4 °C stehengelassen. Der ausgefallene N,N'-Dicyclohexylharnstoff wird durch Filtrieren entfernt und das Filtrat wird mit Natriumbicarbonatlösung und Kohle gewaschen, getrocknet und eingedampft. Durch Rekristallisation des Rückstandes aus Methanol erhält man 5,1 g (42 %) $N^{\alpha}$-(1-Adamantylsulfonyl)-$N^{\varepsilon}$-phthaloyl-L-lysyl-L-alanyl-L-valinal-dimethylacetal vom Schmelzpunkt 214-217 °C.

Analyse für $C_{34}H_{50}N_{40}O_8S$ (674,86) :
Berechnet : C 60,51 H 7,47 N 8,30 %
Gefunden : C 60,52 H 7,40 N 8,54 %.

Beispiel 6

(A) 2,2 g (0,008 6 Mol) Monobenzylterephthalat in 40 ml Dimethoxyäthan/Dimethylformamid (1 : 1) werden auf 0 °C gekühlt. 0,99 g (0,008 6 Mol) N-Hydroxysuccinimid und 1,94 g (0,009 4 Mol) N,N'-Dicyclohexylcarbodiimid werden zugegeben und das Gemisch wird während 3 Stunden bei 0 °C gerührt. Das Acetatsalz des Peptidacetals [hergestellt aus 4 g (0,005 7 Mol) $N^{\alpha}$-(1-Adamantylsulfonyl)-N-phthaloyl-L-lysyl-L-valinal-dimethylacetal wie in Beispiel 1(A) beschrieben] wird in Dimethoxyäthan gelöst, auf 0 °C gekühlt, mit 1,0 ml Triäthylamin neutralisiert und dem oben beschriebenen aktiven Ester zugegeben. Das Gemisch wird während 1 Stunde bei 0 °C gerührt und dann über Nacht bei Raumtemperatur stehengelassen. Die Lösung wird filtriert und das Filtrat wird eingedampft. Der Rückstand wird in Essigester gelöst, mit Wasser und Natriumbicarbonatlösung gewaschen, über Magnesiumsulfat getrocknet und zu einem Schaum eingedampft. Durch Chromatographie an 70 g Silicagel unter Verwendung von Essigester als Eluiermittel erhält man 2,5 g (54 %) $N^{\alpha}$-(1-Adamantylsulfonyl)-$N^{\varepsilon}$-(4-benzyloxycarbonylbenzoyl)-L-lysyl-L-prolyl-L-valinal-dimethylacetal in Form eines Schaums.

Analyse für $C_{43}H_{60}N_4O_9S$ (809,04) :
Berechnet : C 63,84 H 7,48 N 6,93 %
Gefunden : C 63,98 H 7,39 N 6,91 %.

(B) 1.3 g (1,6 mMol) $N^{\alpha}$-(1-Adamantylsulfonyl)-$N^{\varepsilon}$-(4-benzyloxycarbonylbenzoyl)-L-lysyl-L-prolyl-L-valinal-dimethyl acetal in 15 ml Dimethylformamid wird während 3 Stunden in Gegenwart von 5 % Palladium-Kohle hydriert. Der Katalysator wird durch Filtrieren entfernt und das Filtrat wird zu einem Oel eingedampft. Das Oel wird in Natriumbicarbonatlösung aufgenommen und mit Essigester extrahiert. Die wässrige Phase wird mit verdünnter Salzsäure auf pH 2-3 angesäuert und mit Essigester extrahiert. Die organischen Phasen werden mit Wasser von der Säure freigewaschen, über Magnesiumsulfat getrocknet und liefern nach Eindampfen einen Schaum. Dieser Schaum wird an 25 g Silicagel unter Verwendung von Dichlormethan/Methanol/Essigsäure (190 : 9 : 2) als Eluiermittel chromatographiert. Durch Eindampfen des Eluates und Verreiben des Rückstandes mit Diäthyläther erhält man 0,35 g (33 %) $N^{\alpha}$-(1-Adamantylsulfonyl)-$N^{\varepsilon}$-(4-carboxybenzoyl)-L-lysyl-L-prolyl-L-valinal in Form eines Feststoffes.

Analyse für $C_{34}H_{48}N_4O_8S$ (672,84) :
Berechnet : C 60,69 H 7,19 N 8,33 %
Gefunden : C 60,51 H 7,49 N 8,60 %.

## Beispiel 7

In analoger Weise zu Beispiel 7, noch unter Verwendung von Monobenzyladipat anstelle von Monobenzylterephthalat, erhält man $N^{\alpha}$-(1-Adamantylsulfonyl)-$N^{\varepsilon}$-adipoyl-L-lysyl-L-prolylvalinal als Epimerengemisch.

Analyse für $C_{32}H_{52}N_4O_8S$ (652,85) :
Berechnet: C 58,87 H 8,03 N 8,58 %
Gefunden : C 57,99 H 7,95 N 8,53 ; $H_2O$ : 1,54 %.
Wasserfrei C 58,89 H 7,89 N 8,65 %.

## Beispiel 8

In analoger Weise zu Beispiel 7, jedoch aus Monobenzylterephthalat und $N^{\alpha}$-(1-Adamantylsulfonyl)-$N^{\varepsilon}$-phthaloyl-L-lysyl-L-alanyl-L-valinal-dimethylacetal erhält man $N^{\alpha}$-(1-Adamantylsulfonyl)-$N^{\varepsilon}$-(4-carboxybenzoyl)-L-lysyl-L-alanyl-L-valinal in Form eines amorphen Feststoffes.

Analyse für $C_{32}H_{46}N_4O_8S$ (646,81) :
Berechnet : C 59,42 H 7,17 N 8,66 %
Gefunden : C 59,38 H 7,07 N 8,73 %.

## Beispiel A

Eine Aerosolzusammensetzung enthält die folgenden Ingredienzien :

| Ingredienz | Prozent bei Gewicht |
|---|---|
| Verbindung der Formel I oder Salz davon | 1-5 |
| Aethanol | 15-35 |
| Treibgas | ad 100 |

Das Treibgas kann z. B. Dichlordifluoromethan oder ein 5 : 1-Gemisch von 1,2-Dichlor-1,1,2,2-tetrafluoräthan und Dichlordifluoromethan sein.

**Patentansprüche** (für die Vertragsstaaten : BE, CH, DE, FR, GB, IT, LI, LU, NL, SE)

1. Verbindungen der allgemeinen Formel

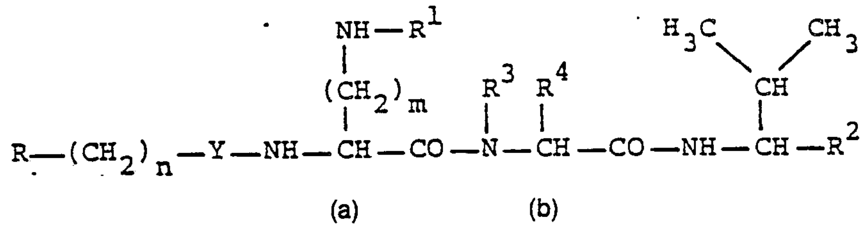

(I)

worin R eine cyclische Kohlenwasserstoffgruppe von 6-12 C-Atomen bedeutet, $R^1$ ein von einer gegebenenfalls durch Amino oder eine oder mehrere Hydroxygruppen substituierten aliphatischen oder aromatischen Dicarbonsäure abgeleiteter Acylrest ist, oder ein Acylrest einer Uronsäure bedeutet, $R^2$ Wasserstoff oder Formyl bedeutet, $R^3$ Wasserstoff und $R^4$ Methyl bedeutet oder $R^3$ und $R^4$ gemeinsam eine Trimethylengruppe bedeuten, Y eine Carbonyl-, Sulfonyl- oder Methylengruppe bedeutet, m für eine ganze Zahl von 2-6 steht und n 0, 1 oder 2 bedeutet, und wobei die mit (a) und (b) bezeichneten C-Atome die L-Konfiguration aufweisen, sowie pharmazeutisch annehmbare Salze davon.

2. Verbindungen nach Anspruch 1, worin $R^1$ eine Acylgruppe bedeutet, die von einer aliphatischen Dicarbonsäure enthaltend bis 6 C-Atomen abgeleitet ist, welche gegebenenfalls durch eine Aminogruppe oder durch eine oder mehrere Hydroxygruppen substituiert ist, oder eine Acylgruppe bedeutet, die von einer Uronsäure abgeleitet ist, $R^3$ und $R^4$ gemeinsam eine Trimethylengruppe bedeutet und sofern $R^2$ eine Formylgruppe bedeutet, die Konfiguration beim C-Atom, an welches diese Formylgruppe angebunden ist, die L-Konfiguration ist.

3. Verbindungen nach Anspruch 1 oder 2, worin R eine monocyclische oder verknüpfte Cycloalkylgruppe enthaltend von 6-12 C-Atomen bedeutet.

4. Verbindungen gemäss einem der Ansprüche 1-3, worin $R^1$ eine Acylgruppe bedeutet, die von einer unsubstituierten gesättigten aliphatischen Dicarbonsäure enthaltend bis 6 C-Atome abgeleitet ist.

5. Verbindungen nach Anspruch 4, worin $R^1$ eine Succinyl- oder Adipoylgruppe bedeutet.

6. Verbindungen nach Anspruch 1 oder 3, worin $R^1$ eine Acylgruppe bedeutet, die von einer unsubstituierten Benzoldicarbonsäure abgeleitet ist.

7. Verbindungen gemäss Anspruch 6, worin $R^1$ eine 4-Carboxybenzoylgruppe bedeutet.

8. Verbindungen nach einem der Ansprüche 1-7, worin $R^2$ eine Formylgruppe bedeutet.

9. Verbindungen nach einem der Ansprüche 1-8, worin Y eine Sulfonylgruppe bedeutet.

10. Verbindungen nach einem der Ansprüche 1-9, worin m für 4 steht.

11. Verbindungen nach einem der Ansprüche 1-10, worin n für 0 steht.

12. $N^{\alpha}$-(1-Adamantylsulfonyl)-$N^{\varepsilon}$-succinyl-L-lysyl-L-prolyl-L-valinal.

13. $N^{\alpha}$-(1-Adamantylsulfonyl)-$N^{\varepsilon}$-(4-carboxybenzoyl)-L-lysyl-L-prolyl-L-valinal.

14. $N^{\alpha}$-(1-Adamantylsulfonyl)-$N^{\varepsilon}$-(4-carboxybenzoyl)-L-lysyl-L-alanyl-L-valinal.

15. Eine Verbindung gemäss Anspruch 1, ausgewählt aus der Gruppe bestehend aus :
$N^{\alpha}$-(1-Adamantyläthyl)-$N^{\varepsilon}$-succinyl-L-lysyl-L-prolinisobutylamid,
$N^{\alpha}$-(6,6-Dimethylbicyclo [3.1.1] heptyläthyl)-$N^{\varepsilon}$-($\gamma$-glutamyl)-L-lysyl-L-prolin-isobutylamid und
$N^{\alpha}$-(1-Adamantanacetyl)-$N^{\varepsilon}$-succinyl-L-lysyl-L-prolinisobutylamid.

16. Eine Verbindung gemäss Anspruch 1, ausgewählt aus der Gruppe bestehend aus :
$N^{\alpha}$-(1-Adamantylsulfonyl)-$N^{\varepsilon}$-succinyl-L-lysyl-L-alanylvalinal,
$N^{\alpha}$-(1-Adamantylsulfonyl)-$N^{\varepsilon}$-(2-carboxybenzoyl)-L-lysyl-L-prolylvalinal und
$N^{\alpha}$-(1-Adamantylsulfonyl)-$N^{\varepsilon}$-adipoyl-L-lysyl-L-prolylvalinal.

17. Verbindungen der allgemeinen Formel

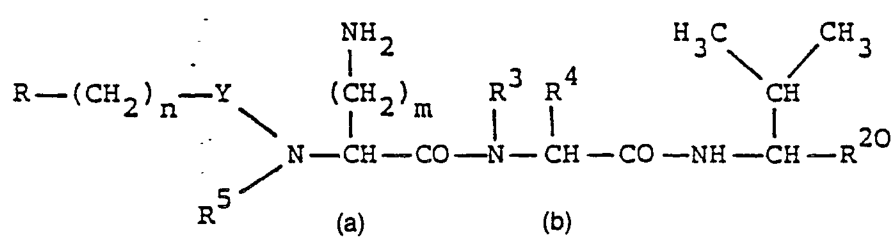

(II)

worin R, $R^3$, $R^4$, Y, m und n die in Anspruch 1 angegebene Bedeutung haben, die mit (a) und (b) bezeichneten Kohlenstoffatomen die Konfiguration gemäss Anspruch 1 haben, $R^5$ Wasserstoff bedeutet, wenn Y eine Carbonyl- oder Sulfonylgruppe bedeutet oder eine Schutzgruppe bedeutet, wenn Y eine Methylengruppe ist und $R^{20}$ ein Wasserstoffatom oder eine geschützte Formylgruppe bedeutet.

18. Verbindungen der allgemeinen Formel

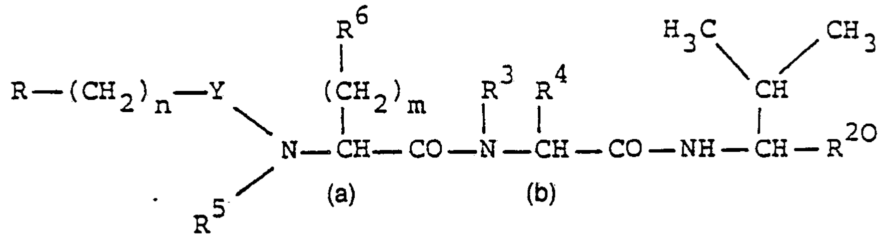

(V)

worin R, $R^3$, $R^4$, Y, m und n die in Anspruch 1 angegebene Bedeutung haben, R und $R^{20}$ die in Anspruch 17 angegebene Bedeutung haben, $R^6$ eine geschützte Aminogruppe bedeutet und die Konfiguration bei den mit (a) und (b) bezeichneten C-Atomen diejenige gemäss Anspruch 1 ist.

19. Eine Verbindung der Formel I gemäss Anspruch 1 oder ein pharmazeutisch annehmbares Salz davon zur Verwendung als pharmazeutisch aktiver Wirkstoff.

20. Eine Verbindung der Formel I gemäss Anspruch 1 oder ein pharmazeutisch annehmbares Salz davon zur Verwendung als pharmazeutisch aktiver Wirkstoff für die Behandlung von degenerativen Erkrankung, die mit der Wirkung von Elastase-ähnlichen Enzymen begleitet sind, oder für die Behandlung von entzündlichen Zuständen, in welchen Elastase-ähnliche Enzyme als Mittler für die Entzündung wirken.

21. Eine Verbindung der Formel I gemäss Anspruch 1 oder ein pharmazeutisch annehmbares Salz davon als pharmazeutischer Wirkstoff für die Behandlung von Emphysemen oder Arthritis.

22. Verfahren zur Herstellung von Verbindungen der Formel I gemäss Anspruch 1 und von pharmazeutisch annehmbaren Salzen davon, dadurch gekennzeichnet, dass man eine Verbindung der allgemeinen Formel

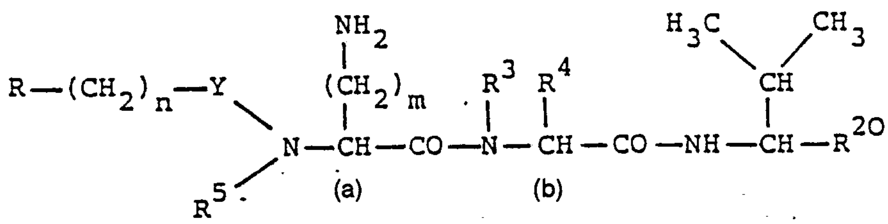

(II)

worin R, $R^3$, $R^4$, Y, m und n die in Anspruch 1 angegebene Bedeutung haben, die Konfiguration bei den mit (a) und (b) bezeichneten C-Atomen diejenige gemäss Anspruch 1 ist, $R^5$ Wasserstoff bedeutet, wenn Y eine Carbonyl- oder Sulfonylgruppe bedeutet oder eine Schutzgruppe bedeutet, wenn Y eine Methylengruppe ist und $R^{20}$ ein Wasserstoffatom oder eine geschützte Formylgruppe bedeutet, mit einem reaktiven Derivat einer Säure der allgemeinen Formel

$$HO—R^{10} \qquad (III)$$

worin $R^{10}$ die Bedeutung von $R^1$ gemäss Anspruch 1 hat, doch worin jede Carboxy-, Amino- oder Hydroxygruppe in geschützter Form vorliegt, umsetzt und, sofern notwendig, die im Reaktionsprodukt vorhandenen Schutzgruppen abspaltet oder

(b) zur Herstellung einer Verbindung der Formel I, in der $R^1$ eine 2-Carboxybenzoylgruppe bedeutet, eine Verbindung der Formel

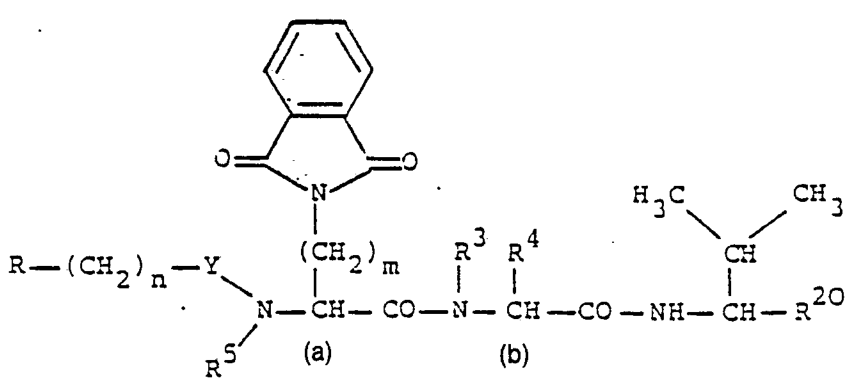

(IV)

worin R, $R^3$, $R^4$, Y, m und n die in Anspruch 1 angegebene Bedeutung haben, $R^5$ und $R^{20}$ die oben erwähnte Bedeutung haben und die Konfiguration bei den mit (a) und (b) bezeichneten C-Atomen

diejenige gemäss Anspruch 1 ist, mit einem Alkalimetallhydroxyd behandelt und, sofern notwendig, allfällige Schutzgruppen im Reaktionsprodukt abspaltet und

(c) in beiden Fällen, sofern erwünscht, eine erhaltene Verbindung der Formel I in ein pharmazeutisch annehmbares Salz überführt.

23. Arzneimittel enthaltend eine Verbindung der Formel I gemäss Anspruch 1 oder ein pharmazeutisch annehmbares Salz davon und einen pharmazeutisch verträglichen Trägerstoff.

24. Arzneimittel für die Behandlung von degenerativen Krankheiten, die mit der Wirkung von Elastase-ähnlichen Enzymen begleitet sind, oder zur Behandlung von entzündlichen Zuständen, in denen Elastase-ähnliche Enzyme als Mittler der Entzündung wirken, enthaltend eine Verbindung der Formel I gemäss Anspruch 1 oder ein pharmazeutisch annehmbares Salz davon und ein pharmazeutisch verträgliches Trägermaterial.

25. Arzneimittel für die Behandlung von Emphysemen oder Arthritis, enthaltend eine Verbindung der Formel I gemäss Anspruch 1 oder ein pharmazeutisch annehmbares Salz davon und ein pharmazeutisch verträgliches Trägermaterial.

**Patentansprüche** (für den Vertragsstaat AT)

1. Verfahren zur Herstellung von Verbindungen der allgemeinen Formel

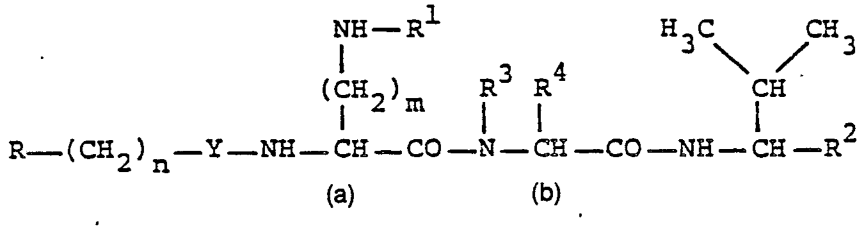

(I)

worin R eine cyclische Kohlenwasserstoffgruppe enthaltend 6-12 C-Atome bedeutet, $R^1$ eine Acylgruppe bedeutet, die von einer aliphatischen oder aromatischen Dicarbonsäure abgeleitet ist, die gegebenenfalls durch eine Amino- oder eine oder mehrere Hydroxygruppen substituiert sein kann, oder eine Acylgruppe bedeutet, die von einer Uronsäure abgeleitet ist, $R^2$ Wasserstoff oder Formyl bedeutet, $R^3$ Wasserstoff bedeutet und $R^4$ Methyl bedeutet oder $R^3$ und $R^4$ zusammen Trimethylen bedeuten, Y Carbonyl, Sulfonyl oder Methylen bedeutet, m für eine ganze Zahl von 2-6 und n für 0, 1 oder 2 steht und worin die Konfiguration bei den mit (a) und (b) bezeichneten C-Atomen die L-Konfiguration ist, und pharmazeutisch annehmbare Salze davon, dadurch gekennzeichnet, dass man eine Verbindung der allgemeinen Formel

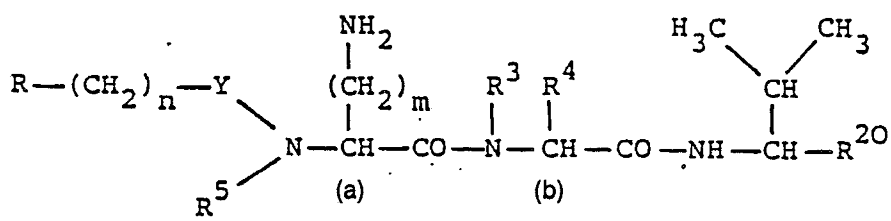

(II)

worin R, $R^3$, $R^4$, Y, m und n die oben erwähnte Bedeutung haben, die Konfiguration bei den mit (a) und (b) bezeichneten C-Atomen die oben erwähnte ist, $R^5$ Wasserstoff bedeutet, wenn Y eine Carbonyl- oder Sulfonylgruppe bedeutet oder eine Schutzgruppe bedeutet, wenn Y eine Methylengruppe ist und $R^{20}$ ein Wasserstoffatom oder eine geschützte Formylgruppe bedeutet, mit einem reaktiven Derivat einer Säure der allgemeinen Formel

$$HO{-}R^{10} \qquad \text{(III)}$$

worin $R^{10}$ die in Anspruch 1 angegebene Bedeutung hat, doch worin jede Carboxy-, Amino- oder Hydroxygruppe in geschützter Form vorliegt, umsetzt und, sofern notwendig, allfällig vorhandene Schutzgruppen entfernt oder

(b) zur Herstellung einer Verbindung der Formel I, worin $R^1$ eine 2-Carboxybenzoylgruppe bedeutet, eine Verbindung der allgemeinen Formel.

(Siehe Formel Seite 17 f.)

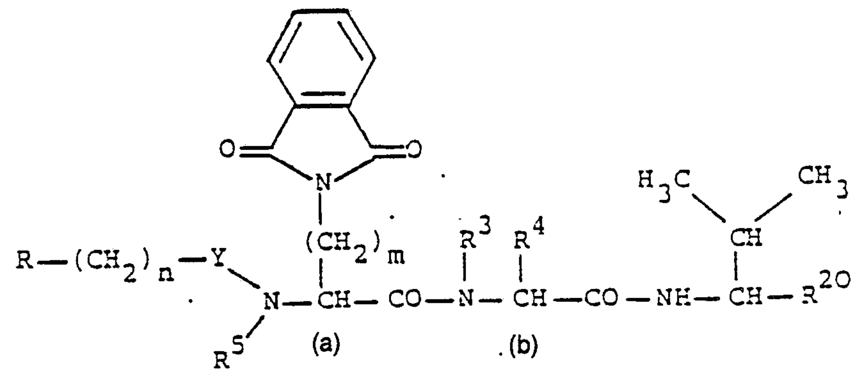

(IV)

worin R, $R^3$, $R^4$, $R^5$, $R^{20}$, Y, m und n die in Anspruch 1 angegebene Bedeutung haben, $R^5$ und $R^{20}$ die oben erwähnte Bedeutung haben und die Konfiguration, bei dem mit (a) und (b) bezeichneten C-Atomen, diejenige gemäss Anspruch 1 ist, mit einem Alkalimetallhydroxyd behandelt, und sofern notwendig, allfällige vorhandene Schurzgruppen im Reaktionsprodukt abspaltet und

(c) in beiden Fällen, sofern erwünscht, eine erhaltene Verbindung der Formel I in ein pharmazeutisch annehmbares Salz überführt.

2. Verfahren nach Anspruch 1 zur Herstellung von Verbindungen der Formel I, worin $R^1$ eine Acylgruppe bedeutet, die von einer aliphatischen Dicarbonsäure enthaltend bis zu 6 C-Atomen abgeleitet ist, die gegebenenfalls durch eine Amino- oder eine oder mehrere Hydroxygruppen substituiert sein kann, oder eine Acylgruppe bedeutet, die von einer Uronsäure abgeleitet ist, $R^3$ und $R^4$ gemeinsam eine Trimethylengruppe bedeuten und, wenn $R^2$ eine Formylgruppe bedeutet, die Konfiguration beim C-Atom, an welches diese Formylgruppe gebunden ist, die L-Konfiguration ist.

3. Verfahren nach Anspruch 1 oder 2 zur Herstellung von Verbindungen der Formel I, worin R eine monocyclische oder verknüpfte Cycloalkylgruppe enthaltend von 6-12 C-Atomen bedeutet.

4. Verfahren nach einem der Ansprüche 1-3 für die Herstellung von Verbindungen der Formel I, worin $R^1$ eine Acylgruppe bedeutet, die von einer unsubstituierten gesättigten aliphatischen Dicarbonsäure enthaltend von bis zu 6 C-Atomen abgeleitet ist.

5. Verfahren nach Anspruch 4 zur Herstellung von Verbindungen der Formel I, worin $R^1$ eine Succinyl- oder Adipoylgruppe bedeutet.

6. Verfahren nach Anspruch 1 oder 3 zur Herstellung von Verbindungen der Formel I, worin $R^1$ eine Acylgruppe bedeutet, die von einer ungesättigten Benzoldicarbonsäure abgeleitet ist.

7. Verfahren nach Anspruch 6 zur Herstellung von Verbindungen der Formel I, worin $R^1$ eine 4-Carboxybenzoylgruppe bedeutet.

8. Verfahren nach einem der Ansprüche 1-7 zur Herstellung von Verbindungen der Formel I, worin $R^2$ eine Formylgruppe bedeutet.

9. Verfahren nach einem der Ansprüche 1-8 zur Herstellung von Verbindungen der Formel I, worin Y eine Sulfonylgruppe bedeutet.

10. Verfahren nach einem der Ansprüche 1-9 zur Herstellung von Verbindungen der Formel I, worin m für 4 steht.

11. Verfahren nach einem der Ansprüche 1-10 zur Herstellung von Verbindungen der Formel I, worin n für 0 steht.

12. Verfahren nach Anspruch 1 zur Herstellung von $N^{\alpha}$-(1-Adamantylsulfonyl)-$N^{\varepsilon}$-succinyl-L-lysyl-L-prolyl-L-valinal.

13. Verfahren nach Anspruch 1 zur Herstellung von $N^{\alpha}$-(1-Adamantylsulfonyl)-$N^{\varepsilon}$-(4-carboxybenzoyl)-L-lysyl-L-propyl-L-valinal.

14. Verfahren nach Anspruch 1 zur Herstellung von $N^{\alpha}$-(1-Adamantylsulfonyl)-$N^{\varepsilon}$-(4-carboxybenzoyl)-L-lysyl-L-alanyl-L-valinal.

15. Verfahren nach Anspruch 1 zur Herstellung von einer Verbindung gemäss Anspruch 1 ausgewählt aus der Gruppe bestehend aus :

$N^{\alpha}$-(1-Adamantyläthyl)-$N^{\varepsilon}$-succinyl-L-lysyl-L-prolinisobutylamid.

$N^{\alpha}$-(6,6-Dimethylbicyclo [3.1.1] heptyläthyl)-$N^{\varepsilon}$-($\gamma$-glutamyl)-L-lysyl-L-prolin-isobutylamid und

$N^{\alpha}$-(1-Adamantanacetyl)-$N^{\varepsilon}$-succinyl-L-lysyl-L-prolinisobutylamid.

16. Verfahren nach Anspruch 1 zur Herstellung von einer Verbindung gemäss Anspruch 1 ausgewählt aus der Gruppe bestehend aus :

$N^{\alpha}$-(1-Adamantylsulfonyl)-$N^{\varepsilon}$-succinyl-L-lysyl-L-alanylvalinal,

$N^{\alpha}$-(1-Adamantylsulfonyl)-$N^{\varepsilon}$-(2-carboxybenzoyl)-L-lysyl-L-prolylvalinal und

$N^{\alpha}$-(1-Adamantylsulfonyl)-$N^{\varepsilon}$-adipoyl-L-lysyl-L-prolylvalinal.

**Claims** (for the Contracting States : BE, CH, DE, FR, GB, IT, LI, LU, NL, SE)

1. Compounds of the general formula

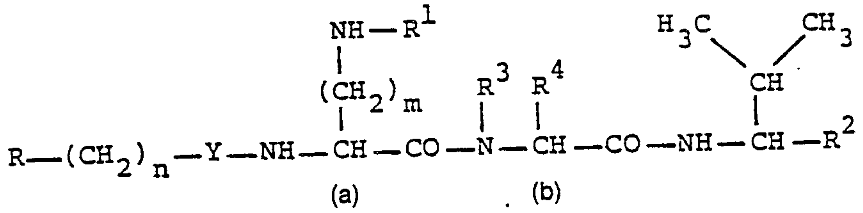

(I)

wherein R signifies a cyclic hydrocarbon group of 6-12 C-atoms, $R^1$ is an acyl residue derived from an aliphatic or aromatic dicarboxylic acid, which is optionally substituted by amino or one or more hydroxy groups, or signifies an acyl residue of a uronic acid, $R^2$ signifies hydrogen or formyl, $R^3$ signifies hydrogen and $R^4$ signifies methyl or $R^3$ and $R^4$ together signify a trimethylene group, Y signifies a carbonyl, sulphonyl or methylene group, m stands for a whole number of 2-6 and n signifies 0, 1 or 2, and wherein the C-atoms designated as (a) and (b) have the L-configuration, as well as pharmaceutically acceptable salts thereof.

2. Compounds according to claim 1, wherein $R^1$ signifies an acyl group which is derived from an aliphatic dicarboxylic acid containing up to 6 C-atoms, which is optionally substituted by an amino group or by one or more hydroxy groups, or signifies an acyl group which is derived from a uronic acid. $R^3$ and $R^4$ together signify a trimethylene group and, insofar as $R^2$ signifies a formyl group, the configuration at the C-atom to which this formyl group is attached is the L-configuration.

3. Compounds according to claim 1 or 2, wherein R signifies a monocyclic or bridged cycloalkyl group containing from 6-12 C-atoms.

4. Compounds in accordance with any one of claims 1-3, wherein $R^1$ signifies an acyl group which is derived from an unsubstituted saturated aliphatic dicarboxylic acid containing up to 6 C-atoms.

5. Compounds according to claim 4, wherein $R^1$ signifies a succinyl or adipoyl group.

6. Compounds according to claim 1 or 3, wherein $R^1$ signifies an acyl group which is derived from an unsubstituted benzenedicarboxylic acid.

7. Compounds in accordance with claim 6, wherein $R^1$ signifies a 4-carboxybenzoyl group.

8. Compounds according to any one of claims 1-7, wherein $R^2$ signifies a formyl group.

9. Compounds according to any one of claims 1-8, wherein Y signifies a sulphonyl group.

10. Compounds according to any one of claims 1-9, wherein m stands for 4.

11. Compounds according to any one of claims 1-10, wherein n stands for 0.

12. $N^\alpha$-(1-Adamantylsulphonyl)-$N^\varepsilon$-succinyl-L-lysyl-L-propyl-L-valinal.

13. $N^\alpha$-(1-Adamantylsulphonyl)-$N^\varepsilon$-(4-carboxybenzoyl)-L-lysyl-L-prolyl-L-valinal.

14. $N^\alpha$-(1-Adamantylsulphonyl)-$N^\varepsilon$-(4-carboxybenzoyl)-L-lysyl-L-alanyl-L-valinal.

15. A compound in accordance with claim 1, selected from the group consisting of:
$N^\alpha$-(1-Adamantylethyl)-$N^\varepsilon$-succinyl-L-lysyl-L-proline isobutylamide.
$N^\alpha$-(6,6-dimethylbicyclo [3.1.1] heptylethyl)-$N^\varepsilon$-($\gamma$-glutamyl)-L-lysyl-L-proline isobutylamide and
$N^\alpha$-(1-adamantaneacetyl)-$N^\varepsilon$-succinyl-L-lysyl-L-proline isobutylamide.

16. A compound in accordance with claim 1, selected from the group consisting of:
$N^\alpha$-(1-Adamantylsulphonyl)-$N^\varepsilon$-succinyl-L-lysyl-L-alanylvalinal.
$N^\alpha$-(1-adamantylsulphonyl)-$N^\varepsilon$-(2-carboxybenzoyl)-L-lysyl-L-prolylvalinal and
$N^\alpha$-(1-adamantylsulphonyl)-$N^\varepsilon$-adipoyl-L-lysyl-L-prolylvalinal.

17. Compounds of the general formula

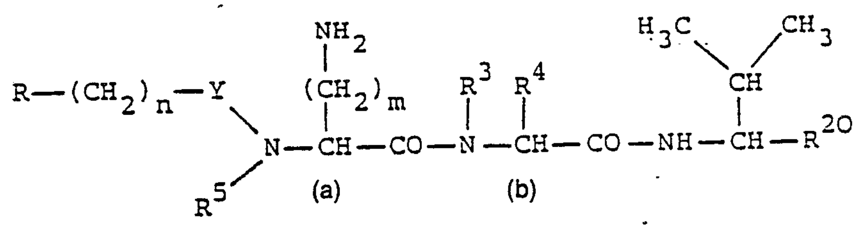

(II)

wherein R, $R^3$, $R^4$, Y, m and n have the significance given in claim 1, the carbon atoms designated as (a) and (b) have the configuration in accordance with claim 1, $R^5$ signifies hydrogen when Y signifies a carbonyl or sulphonyl group or signifies a protecting group when Y is a methylene group and $R^{20}$ signifies a hydrogen atom or a protected formyl group.

18. Compounds of the general formula

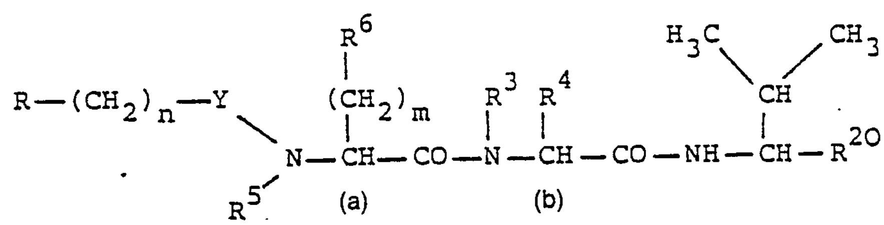

(V)

wherein R, $R^3$, $R^4$, Y, m and n have the significance given in claim 1, R and $R^{20}$ have the significance given in claim 17, $R^6$ signifies a protected amino group and the configuration at the C-atoms designated as (a) and (b) is that in accordance with claim 1.

19. A compound of formula I in accordance with claim 1 or a pharmaceutically acceptable salt thereof for use as a pharmaceutically active substance.

20. A compound of formula I in accordance with claim 1 or a pharmaceutically acceptable salt thereof for use as a pharmaceutically active substance for the treatment of degenerative diseases which are associated with the action of elastase-like enzymes or for the treatment of inflammatory conditions in which elastaselike enzymes act as mediators of inflammation.

21. A compound of formula I in accordance with claim 1 or a pharmaceutically acceptable salt thereof as a pharmaceutically active substance for the treatment of emphysema or arthritis.

22. A process for the manufacture of compounds of formula I in accordance with claim 1 and of pharmaceutically acceptable salts thereof, characterized by reacting a compound of the general formula

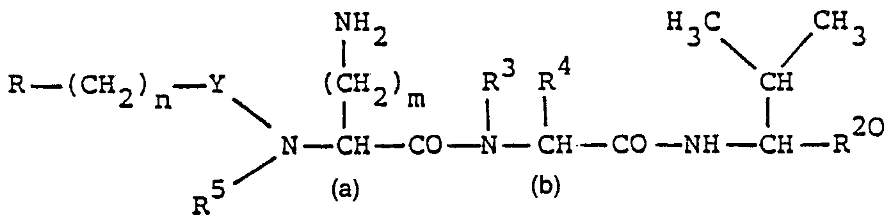

(II)

wherein R, $R^3$, $R^4$, Y, m and n have the significance given in claim 1, the configuration at the C-atoms designated as (a) and (b) is that in accordance with claim 1, $R^5$ signifies hydrogen when Y signifies a carbonyl or sulphonyl group or signifies a protecting group when Y is a methylene group and $R^{20}$ signifies a hydrogen atom or a protected formyl group, with a reactive derivative of an acid of the general formula

$$HO{-}R^{10} \qquad \text{(III)}$$

wherein $R^{10}$ has the significance of $R^1$ in accordance with claim 1, but in which any carboxy, amino or hydroxy group is present in protected form, and, where required, cleaving off the protecting groups present in the reaction product, or

(b) for the manufacture of a compound of formula I in which $R^1$ signifies a 2-carboxybenzoyl group, treating a compound of the formula

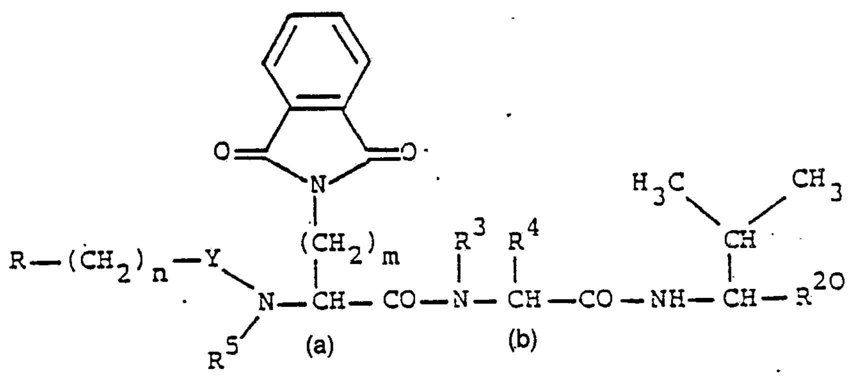

(IV)

wherein R, $R^3$, $R^4$, Y, m and n have the significance given in claim 1, $R^5$ and $R^{20}$ have the significance mentioned above and the configuration at the C-atoms designated as (a) and (b) is that in accordance with claim 1, with an alkali metal hydroxide and, where required, cleaving off any protecting groups in the reaction product, and

(c) in both cases, if desired, converting a compound of formula I obtained into a pharmaceutically acceptable salt.

23. A medicament containing a compound of formula I in accordance with claim 1 or a pharmaceutically acceptable salt thereof and a pharmaceutically compatible carrier material.

24. A medicament for the treatment of degenerative diseases which are associated with the action of elastaselike enzymes or for the treatment of inflammatory conditions in which elastase-like enzymes act as mediators of inflammation, containing a compound of formula I in accordance with claim 1 or a pharmaceutically acceptable salt thereof and a pharmaceutically compatible carrier material.

25. A medicament for the treatment of emphysema or arthritis, containing a compound of formula I in

accordance with claim 1 or a pharmaceutically acceptable salt thereof and a pharmaceutically compatible carrier material.

**Claims** (for the Contracting State AT)

1. A process for the manufacture of compounds of the general formula

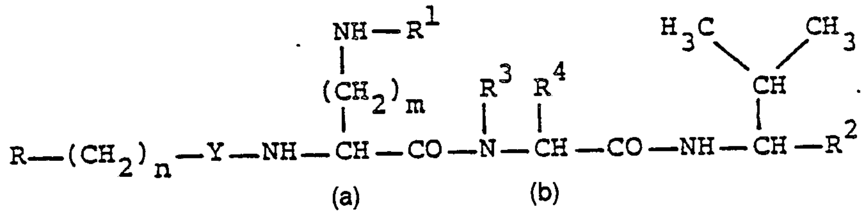
(I)

wherein R signifies a cyclic hydrocarbon group containing 6-12 C-atoms, $R^1$ signifies an acyl group which is derived from an aliphatic or aromatic dicarboxylic acid, which optionally can be substituted by an amino group or one or more hydroxy groups, or signifies an acyl group which is derived from a uronic acid, $R^2$ signifies hydrogen or formyl, $R^3$ signifies hydrogen and $R^4$ signifies methyl or $R^3$ and $R^4$ together signify trimethylene, Y signifies carbonyl, sulphonyl or methylene, m stands for a whole number of 2-6 and n stands for 0, 1 or 2 and wherein the configuration at the C-atoms designated as (a) and (b) is the L-configuration, and pharmaceutically acceptable salts thereof, characterized by reacting a compound of the general formula

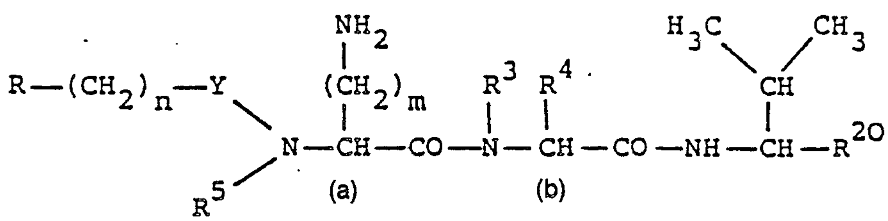
(II)

wherein R, $R^3$, $R^4$, Y, m and n have the significance mentioned above, the configuration at the C-atoms designated as (a) and (b) is that mentioned above, $R^5$ signifies hydrogen when Y signifies a carbonyl or sulphonyl group or signifies a protecting group when Y is a methylene group and $R^{20}$ signifies a hydrogen atom or a protected formyl group, with a reactive derivative of an acid of the general formula

$$HO—R^{10} \quad \text{(III)}$$

wherein $R^{10}$ has the significance given in claim 1, but in which any carboxy, amino or hydroxy group is present in protected form, and, where required, removing any protecting groups present, or

(b) for the manufacture of a compound of formula I in which $R^1$ signifies a 2-carboxybenzoyl group, treating a compound of the general formula

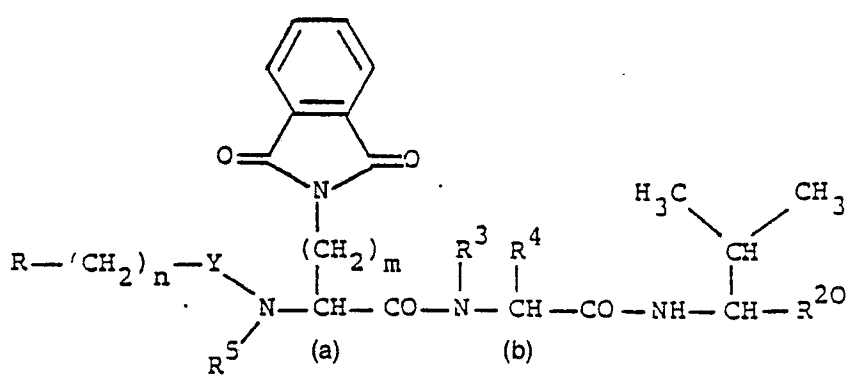
(IV)

wherein R, $R^3$, $R^4$, $R^5$, $R^{20}$, Y, m and n have the significance given in claim 1, $R^5$ and $R^{20}$ have the significance mentioned above and the configuration at the C-atoms designated as (a) and (b) is that in accordance with claim 1, with an alkali metal hydroxide and, where required, cleaving off any protecting groups present in the reaction product, and

(c) in both cases, if desired, converting a compound of formula I obtained into a pharmaceutically acceptable salt.

2. A process according to claim 1 for the manufacture of compounds of formula I in which $R^1$ signifies an acyl group which is derived from an aliphatic dicarboxylic acid containing up to 6 C-atoms, which optionally can be substituted by an amino group or one or more hydroxy groups, or an acyl group which is derived from a uronic acid, $R^3$ and $R^4$ together signify a trimethylene group and, when $R^2$ signifies a formyl group, the configuration at the C-atom to which this formyl group is attached is the L-configuration.

3. A process according to claim 1 or 2 for the manufacture of compounds of formula I in which R signifies a monocyclic or bridged cycloalkyl group containing from 6-12 C-atoms.

4. A process according to any one of claims 1-3 for the manufacture of compounds of formula I in which $R^1$ signifies an acyl group which is derived from an unsubstituted saturated aliphatic dicarboxylic acid containing up to 6 C-atoms.

5. A process according to claim 4 for the manufacture of compounds of formula I in which $R^1$ signifies a succinyl or adipoyl group.

6. A process according to claim 1 or 3 for the manufacture of compounds of formula I in which $R^1$ signifies an acyl group which is derived from an unsaturated benzenedicarboxylic acid.

7. A process according to claim 6 for the manufacture of compounds of formula I in which $R^1$ signifies a 4-carboxybenzoyl group.

8. A process according to any one of claims 1-7 for the manufacture of compounds of formula I in which $R^2$ signifies a formyl group.

9. A process according to any one of claims 1-8 for the manufacture of compounds of formula I in which Y signifies a sulphonyl group.

10. A process according to any one of claims 1-9 for the manufacture of compounds of formula I in which m stands for 4.

11. A process according to any one of claims 1-10 for the manufacture of compounds of formula I in which n stands for 0.

12. A process according to claim 1 for the manufacture of $N^{\alpha}$-(1-adamantylsulphonyl)-$N^{\varepsilon}$-succinyl-L-lysyl-L-prolyl-L-valinal.

13. A process according to claim 1 for the manufacture of $N^{\alpha}$-(1-adamantylsulphonyl)-$N^{\varepsilon}$-(4-carboxybenzoyl)-L-lysyl-L-prolyl-L-valinal.

14. A process according to claim 1 for the manufacture of $N^{\alpha}$-(1-adamantylsulphonyl)-$N^{\varepsilon}$-(4-carboxybenzoyl)-L-lysyl-L-alanyl-L-valinal.

15. A process according to claim 1 for the manufacture of a compound in accordance with claim 1 selected from the group consisting of:

$N^{\alpha}$-(1-Adamantylethyl)-$N^{\varepsilon}$-succinyl-L-lysyl-L-proline isobutylamide.

$N^{\alpha}$-(6,6-dimethylbicyclo [3.1.1] heptylethyl)-$N^{\varepsilon}$-($\gamma$-glutamyl)-L-lysyl-L-proline isobutylamide and

$N^{\alpha}$-(1-adamantaneacetyl)-$N^{\varepsilon}$-succinyl-L-lysyl-L-proline isobutylamide.

16. A process according to claim 1 for the manufacture of a compound in accordance with claim 1 selected from the group consisting of:

$N^{\alpha}$-(1-Adamantylsulphonyl)-$N^{\varepsilon}$-succinyl-L-lysyl-L-alanylvalinal.

$N^{\alpha}$-(1-adamantylsulphonyl)-$N^{\varepsilon}$-(2-carboxybenzoyl)-L-lysyl-L-prolylvalinal and

$N^{\alpha}$-(1-adamantylsulphonyl)-$N^{\varepsilon}$-adipoyl-L-lysyl-L-prolylvalinal.

**Revendications** (pour les Etats contractants : BE, CH, DE, FR, GB, IT, LI, LU, NL, SE)

1. Composés de formule générale

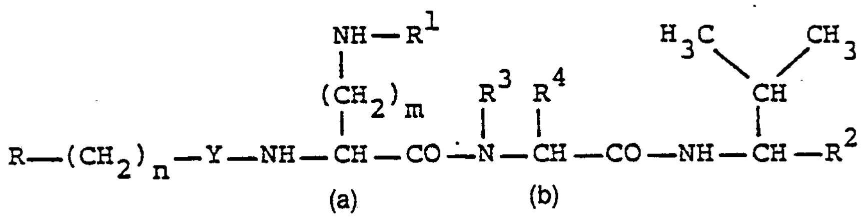

(I)

où R représente un groupe hydrocarboné cyclique en C6 à C12, R1 est un radical acyle dérivé d'un acide dicarboxylique aliphatique ou aromatique éventuellement substitué par un amino ou un ou plusieurs groupes hydroxy, ou un radical acyle d'un acide uronique, R2 représente un hydrogène ou un formyle, R3 représente un hydrogène et R4 un méthyle, ou R3 et R4 représentent ensemble un groupe triméthylène, Y représente un groupe carbonyle, sulfonyle ou méthylène, m représente un nombre entier allant de 2 à 6 et n vaut 0, 1 ou 2, et où les atomes de carbone désignés par (a) et (b) représentent des atomes de carbone et présentent la configuration L, ainsi que leurs sels pharmaceutiquement acceptables.

2. Composés selon la revendication 1, où R1 représente un groupe acyle qui est dérivé d'un acide dicarboxylique aliphatique contenant jusqu'à 6 atomes de carbone, qui est éventuellement substitué par un groupe amino ou par un ou plusieurs groupes hydroxy, ou un groupe acyle qui est dérivé d'un acide uronique, R3 et R4 représentent ensemble un groupe triméthylène et dans la mesure où R2 représente un groupe formyle, la configuration à l'atome de carbone auquel ce groupe formyle est lié est la configuration L.

3. Composés selon la revendication 1 ou 2, où R représente un groupe cycloalcoyle monocyclique ou joint contenant de 6 à 12 atomes de carbone.

4. Composés selon l'une des revendications 1-3, où R1 représente un groupe acyle qui est dérivé d'un acide dicarboxylique aliphatique saturé non substitué contenant jusqu'à 6 atomes de carbone.

5. Composés selon la revendication 4, où R1 représente un groupe succinyle ou adipoyle.

6. Composés selon la revendication 1 ou 3, où R1 représente un groupe acyle qui est dérivé d'un acide benzènedicarboxylique non substitué.

7. Composés selon la revendication 6 où R1 représente un groupe 4-carboxybenzoyle.

8. Composés selon l'une des revendications 1-7, où R2 représente un groupe formyle.

9. Composés selon l'une des revendications 1-8, où Y représente un groupe sulfonyle.

10. Composés selon l'une des revendications 1-9, où m vaut 4.

11. Composés selon l'une des revendications 1-10, où n vaut 0.

12. $N^{\alpha}$-(1-adamantylsulfonyl)-$N^{\varepsilon}$-succinyl-L-lysyl-L-prolyl-L-valinal.

13. $N^{\alpha}$-(1-adamantylsulfonyl)-$N^{\varepsilon}$-(4-carboxybenzoyl)-L-lysyl-L-prolyl-L-valinal.

14. $N^{\alpha}$-(1-adamantylsulfonyl)-$N^{\varepsilon}$-(4-carboxybenzoyl)-L-lysyl-L-alanyl-L-valinal.

15. Composé selon la revendication 1, choisi dans le groupe constitué par :
$N^{\alpha}$-(1-adamantyléthyl)-$N^{\varepsilon}$-succinyl-L-lysyl-L-proline-isobutylamide.
$N^{\alpha}$-(6,6-diméthylbicyclo [3.1.1] heptyléthyl)-$N^{\varepsilon}$-(γ-glutamyl)-L-lysyl-L-proline-isobutylamide et
$N^{\alpha}$-(1-adamantanacétyl)-$N^{\varepsilon}$-succinyl-L-lysyl-L-proline-isobutylamide.

16. Composé selon la revendication 1, choisi dans le groupe constitué par :
$N^{\alpha}$-(1-adamantylsulfonyl)-$N^{\varepsilon}$-succinyl-L-lysyl-L-alanylvalinal,
$N^{\alpha}$-(1-adamantylsulfonyl)-$N^{\varepsilon}$-(2-carboxybenzoyl)-L-lysyl-L-prolylvalinal et
$N^{\alpha}$-(1-adamantylsulfonyl)-$N^{\varepsilon}$-adipoyl-L-lysyl-L-prolylvalinal.

17. Composés de formule générale

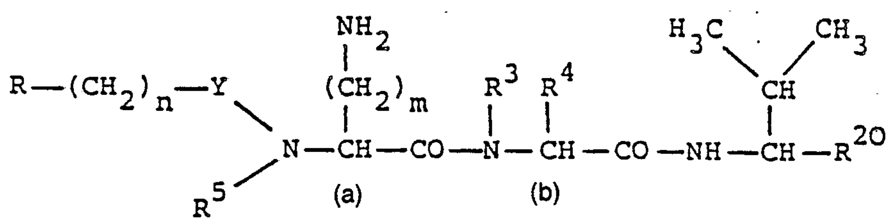

(II)

où R, R3, R4, Y, m et n ont la signification donnée dans la revendication 1, les atomes de carbone désignés par (a) et (b) ont la configuration selon la revendication 1, R5 représente un hydrogène lorsque Y représente un groupe carbonyle ou sulfonyle ou un groupe protecteur lorsque Y est un groupe méthylène et R20 est un atome d'hydrogène ou un groupe formyle protégé.

18. Composés de formule générale

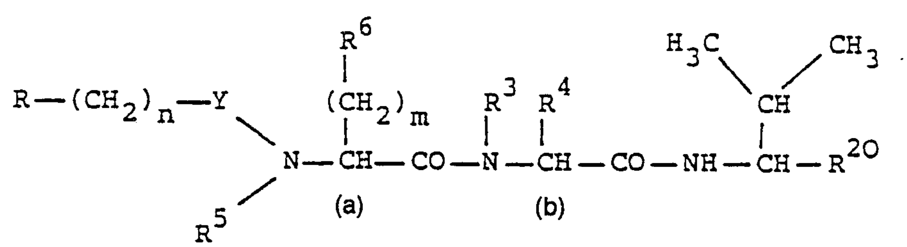

(V)

où R, R3, R4, Y, m et n ont la signification donnée dans la revendication 1, R et R20 ont la signification donnée dans la revendication 17, R6 représente un groupe amino protégé et la configuration dans les atomes de carbone désignés par (a) et (b) est la configuration selon la revendication 1.

19. Composé de formule I selon la revendication 1 ou un de ses sels pharmaceutiquement acceptables aux fins d'application comme substance active pharmaceutique.

20. Composé de formule I selon la revendication 1 ou un de ses sels pharmaceutiquement acceptables aux fins d'application comme substance active pharmaceutique pour le traitement des maladies dégénératives qui s'accompagnent d'une action des enzymes analogues à l'élastase, ou pour le

traitement des états inflammatoires dans lesquels des enzymes similaires à l'élastase agissent comme médiateurs de l'inflammation.

21. Composé de formule I selon la revendication 1 ou un de ses sels pharmaceutiquement acceptables comme substance active pharmaceutique pour le traitement de l'emphysème ou de l'arthrite.

22. Procédé de préparation de composés de formule I selon la revendication 1 et de leurs sels pharmaceutiquement acceptables, caractérisé en ce qu'on fait réagir un composé de formule générale

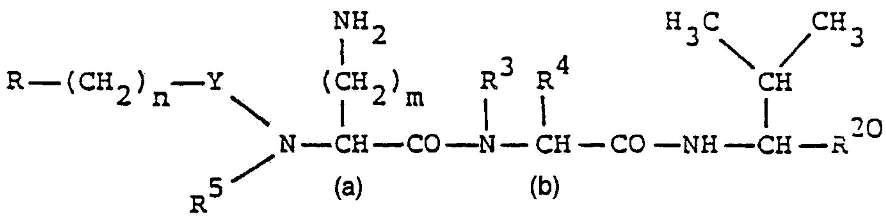

(II)

où R, R3, R4, Y, m et n ont la signification donnée dans la revendication 1, la configuration dans les atomes de carbone désignés par (a) et (b) est celle selon la revendication 1, R5 représente un hydrogène lorsque Y représente un groupe carbonyle ou sulfonyle, ou un groupe protecteur lorsque Y est un groupe méthylène et R20 est un atome d'hydrogène ou un groupe formyle protégé, avec un dérivé réactif d'un acide de formule générale

$$HO—R^{10} \qquad (III)$$

où R10 a la signification de R1 selon la revendication 1, où cependant chaque groupe carboxy, amino ou hydroxy se présente sous forme protégée, et, si nécessaire, on sépare les groupes protecteurs présents dans le produit de la réaction ou

(b) pour préparer un composé de formule I où R1 représente un groupe 2-carboxybenzoyle, on traite un composé de formule

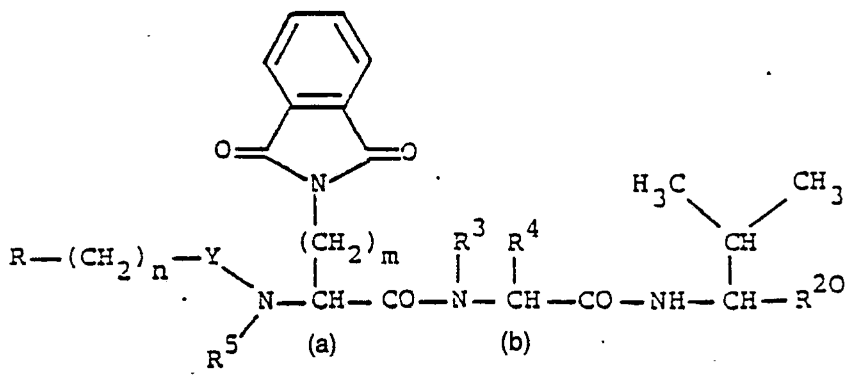

(IV)

où R, R3, R4, Y, m et n ont la signification donnée dans la revendication 1, R5 et R20 ont la signification mentionnée ci-dessus et la configuration dans les atomes de carbone décrits par (a) et (b) est celle selon la revendication 1, avec un hydroxyde de métal alcalin et, si nécessaire, on sépare les groupes protecteurs éventuellement présents dans le produit de la réaction et

(c) dans les deux cas, si on le désire, on transforme un composé de formule I obtenu en un sel pharmaceutiquement acceptable.

23. Médicament contenant un composé de formule I selon la revendication 1 ou un de ses sels pharmaceutiquement acceptables et un support pharmaceutiquement acceptable.

24. Médicament pour le traitement des maladies dégénératives qui s'accompagnent de l'action d'enzymes similaires à l'élastase, ou pour le traitement des états inflammatoires dans lesquels des enzymes similaires à l'élastase agissent comme médiateurs de l'inflammation, contenant un composé de formule I selon la revendication 1 ou un de ses sels pharmaceutiquement acceptables et un support pharmaceutiquement acceptable.

25. Médicament pour le traitement de l'emphysème ou de l'arthrite, contenant un composé de formule I selon la revendication 1 ou un de ses sels pharmaceutiquement acceptables et un support pharmaceutiquement acceptable.

**Revendications** (pour l'Etat contractant AT)

1. Procédé de préparation de composés de formule générale

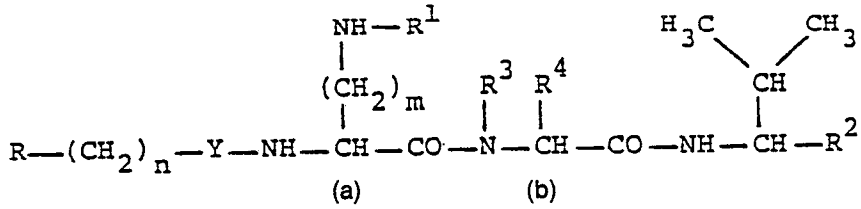

(I)

où R représente un groupe hydrocarboné cyclique en C6 à C12, R1 est un radical acyle dérivé d'un acide dicarboxylique aliphatique ou aromatique éventuellement substitué par un amino ou un ou plusieurs groupes hydroxy, ou un radical acyle d'un acide uronique, R2 représente un hydrogène ou un formyle, R3 représente un hydrogène et R4 un méthyle, ou R3 et R4 représentent ensemble un groupe triméthylène, Y représente un groupe carbonyle, sulfonyle ou méthylène, m représente un nombre entier allant de 2 à 6 et n vaut 0, 1 ou 2, et où les atomes de carbone désignés par (a) et (b) représentent des atomes de carbone et présentent la configuration L, et de leurs sels pharmaceutiquement acceptables, caractérisé en ce qu'on fait réagir un composé de formule générale

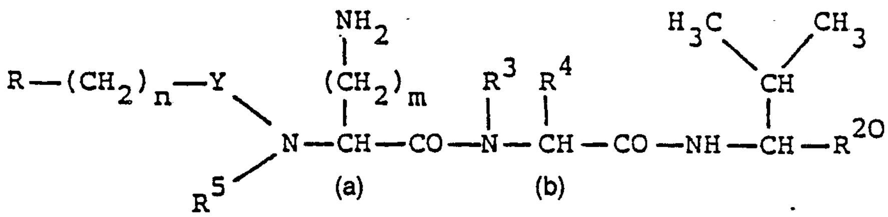

(II)

où R, R3, R4, Y, m et n ont la signification donnée dans la revendication 1, la configuration dans les atomes de carbone désignés par (a) et (b) est celle selon la revendication 1, R5 représente un hydrogène lorsque Y représente un groupe carbonyle ou sulfonyle, ou un groupe protecteur lorsque Y est un groupe méthylène et R20 est un atome d'hydrogène ou un groupe formyle protégé, avec un dérivé réactif d'un acide de formule générale

$$HO—R^{10} \qquad (III)$$

où R10 a la signification de R1 selon la revendication 1, où cependant chaque groupe carboxy, amino ou hydroxy se présente sous forme protégée, et, si nécessaire, on sépare les groupes protecteurs présents dans le produit de la réaction ou

(b) pour préparer un composé de formule I où R1 représente un groupe 2-carboxybenzoyle, on traite un composé de formule

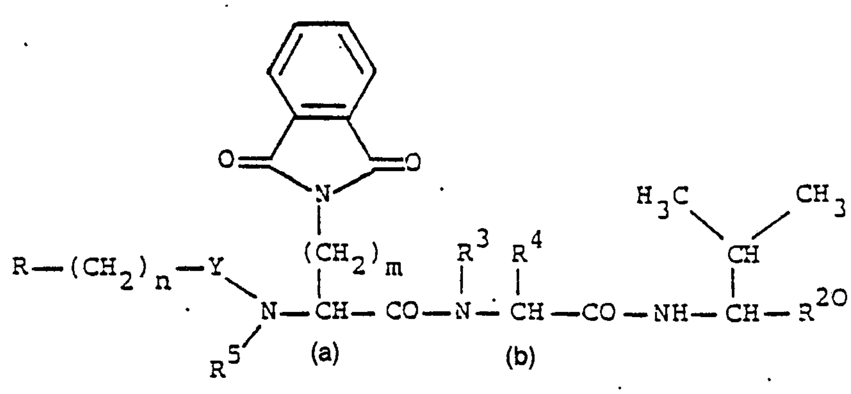

(IV)

où R, R3, R4, Y, m et n ont la signification donnée dans la revendication 1, R5 et R20 ont la signification mentionnée ci-dessus et la configuration dans les atomes de carbone décrits par (a) et (b) est celle selon la revendication 1, avec un hydroxyde de métal alcalin et, si nécessaire, on sépare les groupes protecteurs éventuellement présents dans le produit de la réaction et

(c) dans les deux cas, si on le désire, on transforme un composé de formule I obtenu en un sel pharmaceutiquement acceptable.

2. Procédé selon la revendication 1 de préparation de composés de formule I où R1 représente un groupe acyle qui est dérivé d'un acide dicarboxylique aliphatique contenant jusqu'à 6 atomes de carbone, qui peut être éventuellement substitué par un groupe amino ou un ou plusieurs groupes hydroxy, ou un groupe acyle qui est dérivé d'un acide uronique, R3 et R4 représentent ensemble un groupe triméthylène

et, lorsque R2 représente un groupe formyle, la configuration à l'atome de carbone auquel ce groupe formyle est lié est la configuration L.

3. Procédé selon la revendication 1 ou 2 de préparation de composés de formule I où R représente un groupe cycloalcoyle monocyclique ou joint contenant de 6 à 12 atomes de carbone.

4. Procédé selon l'une des revendications 1-3 de préparation de composés de formule I où R1 représente un groupe acyle qui dérive d'un acide dicarboxylique aliphatique saturé non substitué contenant jusqu'à 6 atomes de carbone.

5. Procédé selon la revendication 4 de préparation de composés de formule I où R1 représente un groupe succinyle ou adipoyle.

6. Procédé selon la revendication 1 ou 3 de préparation de composés de formule I où R1 représente un groupe acyle qui est dérivé d'un acide benzène-dicarboxylique non saturé.

7. Procédé selon la revendication 6 de préparation de composés de formule I où R1 représente un groupe 4-carboxybenzoyle.

8. Procédé selon l'une des revendications 1-7 de préparation de composés de formule I où R2 représente un groupe formyle.

9. Procédé selon l'une des revendications 1-8 de préparation de composés de formule I où Y représente un groupe sulfonyle.

10. Procédé selon l'une des revendications 1-9 de préparation de composés de formule I où m vaut 4.

11. Procédé selon l'une des revendications 1-10 de préparation de composés de formule I où n vaut 0.

12. Procédé selon la revendication 1 de préparation de $N^{\alpha}$-(1-adamantylsulfonyl)-$N^{\varepsilon}$-succinyl-L-lysyl-L-prolyl-L-valinal.

13. Procédé selon la revendication 1 de préparation de $N^{\alpha}$-(1-adamantylsulfonyl)-$N^{\varepsilon}$-(4-carboxybenzoyl)-L-lysyl-L-prolyl-L-valinal.

14. Procédé selon la revendication 1 de préparation de $N^{\alpha}$-(1-adamantylsulfonyl)-$N^{\varepsilon}$-(4-carboxybenzoyl)-L-lysyl-L-alanyl-L-valinal.

15. Procédé selon la revendication 1 de préparation d'un composé selon la revendication 1 choisi dans le groupe constitué par :

$N^{\alpha}$-(1-adamantyléthyl)-$N^{\varepsilon}$-succinyl-L-lysyl-L-proline-isobutylamide,
$N^{\alpha}$-(6,6-diméthylbicyclo [3.1.1] heptyléthyl)-$N^{\varepsilon}$-(γ-glutamyl)-L-lysyl-L-proline-isobutylamide et
$N^{\alpha}$-(1-adamantanacétyl)-$N^{\varepsilon}$-succinyl-L-lysyl-L-proline-isobutylamide.

16. Procédé selon la revendication 1 de préparation d'un composé selon la revendication 1 choisi dans le groupe constitué par :

$N^{\alpha}$-(1-adamantylsulfonyl)-$N^{\varepsilon}$-succinyl-L-lysyl-L-alanylvalinal,
$N^{\alpha}$-(1-adamantylsulfonyl)-$N^{\varepsilon}$-(2-carboxybenzoyl)-L-lysyl-L-prolylvalinal et
$N^{\alpha}$-(1-adamantylsulfonyl)-$N^{\varepsilon}$-adipoyl-L-lysyl-L-prolyl-valinal.